# EUROPÄISCHE PATENTANMELDUNG

(11) **EP 2 471 806 A2**
(43) Veröffentlichungstag der Anmeldung: **04.07.2012**
(21) Anmeldenummer: 12161102.4
(22) Anmeldetag: 17.08.2010
(51) Int. Cl.: C07K 7/64, A61K 38/13

(54) **Cyclosporinderivate**

(30) Priorität: 17.08.2009 DE 102009037551
(62) Teilanmeldung aus: 10745566.9
(71) Anmelder: Max-Planck-Gesellschaft zur Förderung der Wissenschaften e.V., 80539 München (DE)
(72) Erfinder: Fischer, Gunter, 06120 Halle (DE); Malesevic, Miroslav, 06108 Halle (DE); Erdmann, Frank, 06114 Halle (DE); Kühling, Jan, 06108 Halle (DE); Bukrinsky, Michael, Potomac, MD Maryland 20854 (US); Constant, Stephanie, Herndon, VA Virginia 20171 (US)
(74) Vertreter: Stolmár Scheele & Partner

(57) **Zusammenfassung**

Die vorliegende Erfindung betrifft ein Cyclosporinderivat mit der allgemeinen Formel (I) 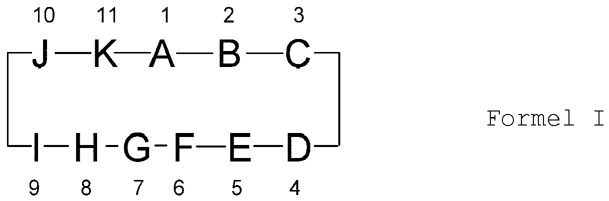 oder ein pharmazeutisch verträgliches Salz davon,
wobei in der Formel (I) A ein Aminosäurerest der allgemeinen Formel (II) 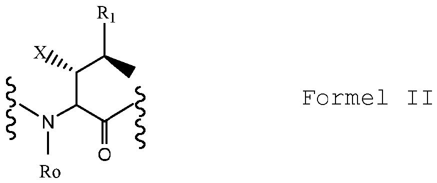 ist und die Reste Rₒ, R₁, B, C, D, E, F, G, H, I, J und K gemäß Anspruch 1 definiert sind.

## Beschreibung

Die vorliegende Erfindung betrifft Cyclosporinderivate, ein Verfahren zur Anreicherung eines Cyclosporinderivats in einem extrazellulären Raum eines multizellularen Objekts, die Verwendung der erfindungsgemäßen Cyclosporinderivate zur Herstellung eines Medikaments und eine pharmazeutische Zusammensetzung, umfassend ein erfindungsgemäßes Cyclosporinderivat.

Biologisch wirksame Moleküle, bei denen es sich in der Regel um pharmazeutische Wirkstoffe handelt, entfalten ihre Wirkung meist sowohl innerhalb wie auch außerhalb von biologischen Zellen. Dabei ist bisher vornehmlich das Problem aufgetreten, dass Wirkstoffe, die ihre Wirkung nur innerhalb der Zelle entfalten sollen, bereits vor Durchtritt durch die Zellmembran unerwünschte Veränderungen im extrazellulären Raum verursachen. Hierbei tritt zusätzlich das Problem auf, dass ein und derselbe Wirkstoff innerhalb und außerhalb der Zelle eine unterschiedliche Wirkung entfalten kann. Die eigentliche Wirkung umfasst somit zwei Komponenten - die erwünschte intrazelluläre und die nicht erwünschte extrazelluläre. Soll die intrazelluläre Wirkung erreicht werden, musste - da der Transport zur Zelle in der Regel die Durchquerung eines extrazellulären Raumes beinhaltet - notwendigerweise oft auch die extrazelluläre (Neben)Wirkung in Kauf genommen werden.

Ein ebenso wichtiges Problem, das im Stand der Technik bisher jedoch nicht beschrieben wurde, ist die Verabreichung von Wirkstoffen, die ihre Wirkung allein außerhalb der Zelle entfalten sollen bzw. dürfen. Vor allem in der Medizin ist eine Reihe von Wirkstoffen bekannt, die in der Zelle nicht nur nicht die beabsichtigte Wirkung entfalten, sondern sogar toxisch wirken bzw. eine anderweitig schädigende Wirkung verursachen. Hinzu kommt, dass zur Erzielung einer bestimmten, extrazellulären Wirkung eine weit höhere Dosis verabreicht werden muss als eigentlich benötigt, um den "Verlust" der in das Zellinnere abgewanderten Wirkstoffe zu kompensieren.

Wirkstoffe können extrazellulär auf Moleküle oder Strukturen wirken. Solche biologischen Moleküle des Extrazellularraumes können beispielsweise Enzyme, Inhibitoren, Aktivatoren, oder Rezeptoren sein. Unter "Strukturen" ist beispielsweise die extrazelluläre Matrix zu verstehen, die aus der Gesamtheit der Makromoleküle, die sich außerhalb der Plasmamembran von Zellen in Geweben und Organen befinden, gebildet ist.

In der gegenwärtigen medizinischen Forschung wichtige Wirkstoffe sind Effektoren, welche entzündliche Prozesse in biologischen Objekten, vorzugsweise in der Tier- und Humanmedizin hemmen können. Effektoren von Peptidyl-Prolylcis/trans-Isomerasen (PPIasen), d.h. PPIase-Inhibitoren, sind dabei von besonderer Bedeutung.

Diese Effektoren können zwar zwischen den einzelnen PPIase-Familien (Nature Chemical Biology. 3(10):619-29, 2007; Cellular & Molecular Life Sciences. 63(24):2889-900, 2006; Current Topics in Medicinal Chemistry. 3(12):1315-47, 2003; Advances in Protein Chemistry. 59:243-82, 2001) unterscheiden, haben aber oft ähnliche Hemmkraft gegenüber Sequenz-ähnlichen Familienmitgliedern. Da PPIasen innerhalb einer Familie unterschiedlichste biochemische Reaktionen beeinflussen können, hängt die diagnostische oder pharmakologische Wirkung verabreichter Wirkstoffe unmittelbar von der erreichten Konzentration in unterschiedlichsten Verteilungsräumen ab. So sind z.B. einige dieser PPIase-Inhibitoren (z.B. Biopolymers 84(2006)125-146; Chemical & Pharmaceutical Bulletin. 54(3):372-376, 2006; Chemistry & Biology. 10(1):15-24, 2003; Nucleic Acids Research. 29(3):767-773, 2001), wie z.B. das therapeutisch verwendete Cyclosporin in Wasser nur schwer löslich. (DE 19859910). Trotzdem werden nach gewöhnlicher medikamentöser Applikation weitaus höhere Konzentrationen innerhalb von Zellen gefunden. Es wird vermutet, dass die Wirkstoffe durch die Zellmembran wandern und sich dann an intrazellulär vorhandene PPIasen binden. Für ein Cyclosporinderivat (SDZ IMM 125) konnten so (Anti-Cancer Drugs. 8(4):400-404, 1997; Journal of Pharmacokinetics & Biopharmaceutics. 22(5):327-65, 1994) in Blutzellen etwa 8x höhere Konzentration als im umgebenden Plasma nachgewiesen werden.

Cyclosporin (auch Ciclosporin) ist ein zyklisches Oligopeptid mit immunosuppressiver und calcineurin-hemmender Wirkung. Es zeichnet sich durch einen selektiven und reversiblen Mechanismus der Immunosuppression aus. Es blockiert selektiv die Aktivierung von T-Lymphozyten über die Produktion von bestimmten Zytokinen, die an der Regulierung dieser T-Zellen beteiligt sind. Dabei wird vor allem die Synthese von Interleukin-2 gehemmt, wodurch gleichzeitig die Proliferation von zytotoxischen T-Lymphozyten, die z.B. für die Abstoßung von fremdem Gewebe verantwortlich sind, supprimiert wird. Cyclosporin wirkt intrazellulär durch Bindung an die so genannten Cyclophiline oder Immunophiline, die zur Familie der Cyclosporin-bindenden Proteine gehören.

Inhibitoren von Cyclophilinen weisen ein sehr großes therapeutisches Spektrum auf, wie z.B. die Behandlung von Erkrankungen des Atmungstraktes, wie z.B. Asthma, COPD, Lungenentzündung oder Emphysem (Expert Opinion on Investigational Drugs 12 (2003), 647-653, Biodrugs 8 (1997) 205-215, American Journal of Respiratory Cell & Molecular Biology 20 (1999), 481-492), Stoffwechselerkrankungen wie Diabetes (Transplantation Proceedings 37 (2005), 1857-1860, Molecular Pharmacology 60 (2001), 873-879), entzündliche Erkrankungen des Verdauungstraktes (Bone Marrow Transplantation 26 (2000), 545-551, Pharmaceutical Research 20 (2003), 910-917), Störungen des Immunsystems (Immunology Letters 84 (2002), 137-143, Acta Biochimica Polonica 49 (2002), 233-247, Entzündungen (Journal of Periodontal Research 42 (2007), 580-588, Journal of Neurology, Neurosurgery & Psychiatry 76 (2005), 1115-1120, Transplant Immunology 12 (2004), 151-157), kardiovaskuläre Erkrankungen (Journal of Hypertension 17 (1999), 1707-1713, Drug & Chemical Toxicology 21 (1998), 27-34), neurologische Erkrankungen (Annals of Vascular Surgery 20 (2006), 243-249), Erkrankungen, die mit einer Störung der Angiogenese einhergehen (Blood Purification 25 (2007), 466-472, International Angiology 24 (2005), 372-379, Nefrologia 23 (2003), 44-48), zur Unterdrückung der Immunantwort bei Organtransplantation (Bone Marrow Transplantation 38 (2006), 169-174), Biodrugs 14 (2000), 185-193, Clinical Immunotherapeutics 5(1996), 351-373) und von Autoimmunerkrankungen (Immunology & Immunopathology 82(3), 197-202, 1997), Erkrankungen des arthritischen Formenkreises (British Journal of Rheumatology 36 (1997), 808-811, Biodrugs 7 (1997), 376-385), Dermatididen (Veterinary Dermatology 17 (2006), 3-16), Psoriasis (Journal of Dermatological Treatment 16 (2005), 258-277, Hautarzt 44 (1993), 353-360), bei Allergien (Cornea 27 (2008), 625, Journal of Small Animal Practice 47 (2006), 434-438, Clinical & Experimental Ophthalmology 34 (2006), 347-353), bei multipler Sklerose (Immunopharmacology & Immunotoxicology 21 (1999), 527-549, Journal of Neuroimaging 7 (1997), 1-7), Erkrankungen, die durch eine Ischämie verursacht werden, wie z.B. Infarkte des Herzens (Annals of Thoracic Surgery 86 (2008), 1286-1292, Acta Anaesthesiologica Scandinavica 51 (2007), 909-913), des Pankreas (Pancreas 32 (2006), 145-151) oder des Hirns (Annals of Vascular Surgery 20 (2006), 243-249, Neurological Research 27 (2005), 827-834), Nierenerkrankungen, wie z.B. Glomerulonephritis (Nephrology Dialysis Transplantation 19 (2004), 3207, Nephron 91 (2002), 509-511), Geschwulste (Journal of Investigative Dermatology 128 (2008), 2467-2473, Endocrinology 148 (2007), 4716-4726), bei multiplen Myelomen (Leukemia 12 (1998), 505-509, Leukemia & Lymphoma 16 (1994), 167-170), bei akuter oder chronischer Leukämie (Cancer Chemotherapy & Pharmacology 52 (2003), 449-452, Cancer 97 (2003), 1481-1487), Muskeldegeneration (Neuroscience Research Communications 31 (2002), 85-92 , Kachexie (International Journal of Cardiology 85 (2002), 173-183, Drugs 58 (1999), 953-963, 1999), Reiter's Syndrom (Rheumatology 40 (2001), 945-947), Knochenabbauerkrankungen (European Journal of Pharmacology 564 (2007), 226-231, Biochemical & Biophysical Research Communications 254 (1999), 248-252), bei der Alzheimer'schen Erkrankung (Biochemical & Biophysical Research Communications 248 (1998), 168-173, Chinese Medical Journal 115 (2002), 884-887), Malaria (Molecular & Biochemical Parasitology 99 (1999), 167-181), dem septischen und toxischen Schock-Syndrom (Journal of Pharmacology & Experimental Therapeutics 311 (2004), 1256-1263), Myalgie (British Journal of Dermatology 147 (2002), 606-607), bei einer Infektion mit Viren (Expert Opinion on Emerging Drugs 13 (2008), 393-416), wie z.B. HIV-1, HIV-2, HIV-3 (Journal of Infectious Diseases 194 (2006), 1677-1685, Molecular Medicine Today 1 (1995), 287-291, 1995), Cytomegaloviren (Journal of Virology 81 (2007), 9013-9023) oder Adenoviren (Ophthalmologe 105 (2008), 592-594, Ophthalmologe 97 (2000), 764-768) und zur Förderung des Haarwachstums (Archives of Dermatological Research 296(6), 265-269, 2004, Annales de Dermatologie et de Venereologie 127 (2000), 769).

Der Komplex aus Cyclosporin und Cyclophilin blockiert anschliessend die Serin-Threonin-Phosphatase Calcineurin. Deren Aktivitätszustand steuert wiederum die Aktivierung von Transkriptionsfaktoren wie etwa NF-KappaB oder NFATp/c, welche bei der Aktivierung verschiedener Zytokin-Gene, darunter auch dasjenige für Interleukin-2, eine entscheidende Rolle spielen. Hierdurch werden die immunkompetenten Lymphozyten während der G0- oder G1-Phase des Zellzyklus arretiert, da die für die Zellteilung essentiellen Proteine wie Interleukin-2 nicht mehr produziert werden können. T-Helfer-Zellen, welche die Aktivität der für die Abstoßung verantwortlichen zytotoxischen T-Zellen erhöhen, sind der bevorzugte Angriffspunkt für Cyclosporin.

Daneben inhibiert Cyclosporin die Synthese und Freisetzung weiterer Lymphokine, die für die Proliferation reifer zytotoxischer T-Lymphozyten und für weitere Funktionen der Lymphozyten zuständig sind. Die Fähigkeit von Cyclosporin, Interleukin-2 zu blockieren, ist für seine klinische Wirksamkeit maßgeblich: Transplantatempfänger, die ihre Transplantate gut tolerieren, zeichnen sich durch eine niedrige Produktion von Interleukin-2 aus. Dagegen ist bei Patienten mit manifester Abstoßungsreaktion keine Hemmung der Interleukin-2-Produktion feststellbar.

Die Aufgabe der vorliegenden Erfindung war es, neue, pharmazeutisch aktive Cyclosporinderivate aufzufinden.

Die Aufgabe der vorliegenden Erfindung wurde gelöst durch ein Cyclosporinderivat der allgemeinen Formel (I) 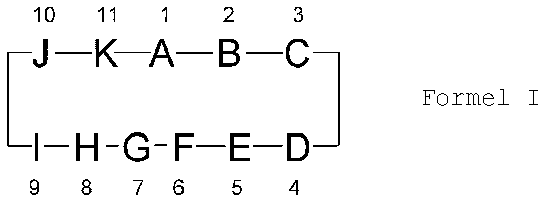 oder ein pharmazeutisch verträgliches Salz davon,
wobei in der Formel (I)
A ein Aminosäurerest der Formel (II) ist, 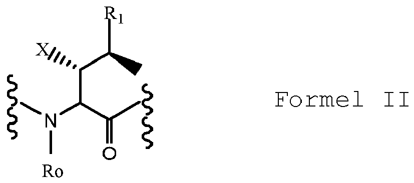 bei dem R₁ einem Rest der Formel III 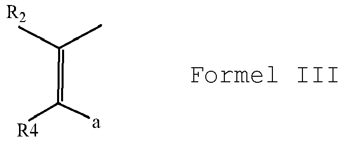 entspricht und über das Kohlenstoffatom a kovalent verknüpft ist, oder
   bei dem R₁ einem Rest der Formel IV 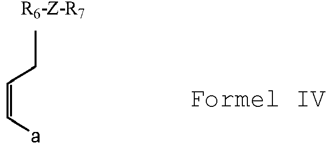 entspricht und über das Kohlenstoffatom a kovalent verknüpft ist, oder
   bei dem R₁ einem Rest der Formel V 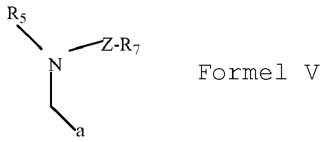 entspricht und über das Kohlenstoffatom a kovalent verknüpft ist, oder bei dem R₁ einem Rest der Formel VI 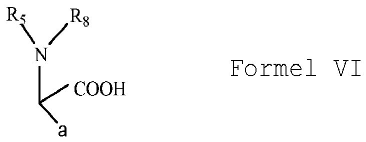 entspricht und über das Kohlenstoffatom a kovalent verknüpft ist, oder
   bei dem R₁ einem Rest der Formel VII 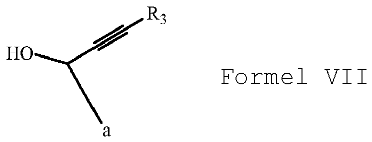 entspricht und über das Kohlenstoffatom a kovalent verknüpft ist, oder
   bei dem R₁ einem Rest der Formel VIII 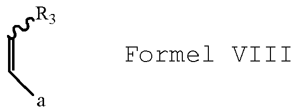 entspricht und über das Kohlenstoffatom a kovalent verknüpft ist, oder
   bei dem R₁ einem Rest der Formel IX entspricht 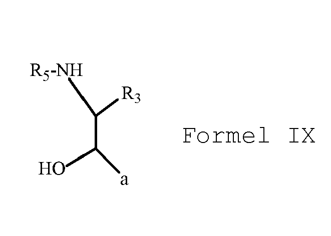 und über das Kohlenstoffatom a kovalent verknüpft ist, wobei die Gruppe C=O in der Formel II über eine Amidbindung kovalent an einer alpha-Aminogruppe von B gebunden ist und die Gruppe N-Rₒ in der Formel II über eine Amidbindung kovalent an einer Carboxylgruppe von K gebunden ist,
B ein Aminosäurerest ist, der aus der Gruppe bestehend aus:
   alpha-Aminobuttersäure,
   Alanin,
   Threonin,
   Valin,
   Norvalin und
   in der Seitenkette mit einer Hydroxylgruppe modifizierter alpha-Aminobuttersäure, Alanin,
   Threonin, Valin oder Norvalin
   ausgewählt ist,
C ein Sarkosinrest ist,
D ein Aminosäurerest ist, der aus der Gruppe bestehend aus:
   Leucin,
   N-Methylleucin,
   Valin,
   gamma-Hydroxy-N-methylleucin und
   gamma-Hydroxyleucin
   ausgewählt ist,
E ein Aminosäurerest ist, der aus der Gruppe bestehend aus:
   Valin,
   Norvalin und
   einem modifizierten Valin oder Norvalin, bei dem ein Kohlenstoffatom der Seitenkette mit einer
   Hydroxylgruppe substituiert ist,
   ausgewählt ist,
F ein Aminosäurerest ist, der aus der Gruppe bestehend aus:
   Leucin,
   N-Methylleucin,
   gamma-Hydroxy-N-methylleucin und
   gamma-Hydroxyleucin
   ausgewählt ist,
G ein alpha-Aminobuttersäurerest oder ein Alaninrest ist,
H ein D-Alaninrest ist,
I und J Reste sind, die unabhängig voneinander aus der Gruppe, bestehend aus:
   Leucin,
   N-Methylleucin,
   gamma-Hydroxy-N-methylleucin und
   gamma-Hydroxyleucin
   ausgewählt sind, und
K N-Methylvalinrest oder Valinrest ist,
   wobei in den Formeln II bis IX
   Rₒ für H oder CH₃ steht,
   X für H oder
   Hydroxyl oder
   eine Hydroxylgruppe steht, die mit einer Alkanoyl-, Aryloyl-, Alkylaminocarbonyl-, Arylaminocarbonyl-, Arylalkylaminocarbonyl-, Alkoxycarbonyl-, Aryloxycarbonyl-, Arylalkyloxycarbonylgruppe oder einer Silylschutzgruppe derivatisiert ist,
   R₂ für H oder einen substituierten oder unsubstituierten monozyklischen, bizyklischen oder trizyklischen Aryl- oder Heteroarylring, der gegebenenfalls mit R₃ substituiert ist, steht,
   R₃ aus der Gruppe bestehend aus:
   - Z-NY-Y₁,
   - Z-CO-Y,
   - Z-S-Y,
   - Y_{2,}
   - NY-Y₁,
   - CO-Y,
   - S-Y,
   - Z-O-Y und
   - O-Y
   ausgewählt ist,
   R₄ der Gruppe R₂ wie vorstehend definiert entspricht, wobei R₄ und R₂ gleich oder verschieden voneinander sein können,
   Z aus der Gruppe bestehend aus:
   einer unverzweigten, verzweigten oder zyklischen Alkylgruppe mit 1 bis 20 Kohlenstoffatomen,
   einer unverzweigten, verzweigten oder zyklischen Alkenylgruppe mit 1 bis 20 Kohlenstoffatomen, einer unverzweigten, verzweigten oder zyklischen Alkinylgruppe mit 1 bis 20 Kohlenstoffatomen, einem substituierten oder unsubstituierten monozyklischen, bizyklischen oder trizyklischen Aryl- oder Heteroarylring und
   einer Gruppe der allgemeinen Formel -[CH₂)ₘ-O-(CH₂)ₙ]ₒ, wobei m, n und O unabhängig voneinander ganze Zahlen von 1 bis 20 sind,
   ausgewählt ist,
   Y aus der Gruppe, bestehend aus: H,
   den geschützten oder ungeschützten Aminosäureresten Gly, D-Glu, D-Asp, D- und L-Cys(SO₃H), ungeschützten Peptidresten mit einer Kettenlänge von 2 bis 200 Aminosäureresten, enthaltend eine
   Aminosäureart oder eine Kombination von verschiedenen Aminosäurearten ausgewählt aus Gly, D-Glu, D-Asp, D-und L-Cys(SO₃H), einer Alkyl-, Alkenyl-, Aryl-, Alkylaminocarbonyl-, Arylaminocarbonyl-, Arylalkylaminocarbonyl-, Alkylcarboxyl-, Alkoxycarbonyl-, Aryloxycarbonyl- und
   Arylalkyloxycarbonylgruppe
   ausgewählt ist,
   Y₁ der Gruppe Y wie vorstehend definiert entspricht, wobei Y und Y₁ gleich oder verschieden voneinander sein können,
   Y₂ für H, F, Cl, Br oder I steht,
   R₅ aus der Gruppe bestehend aus:
   einer Alkyl-, Alkenyl-, Aryl-, Alkylaminocarbonyl-, Arylaminocarbonyl-, Arylalkylaminocarbonyl-, Alkylcarbonyl-, Alkoxycarbonyl-, Aryloxycarbonyl-, Arylalkyloxycarbonylgruppe,
   den Aminosäureresten Gly, D-Glu, D-Asp, D- und L-Cys(SO₃H),
   Peptidresten mit einer Kettenlänge von 2 bis 200 Aminosäureresten, enthaltend eine Aminosäureart oder eine Kombination von verschiedenen Aminosäurarten ausgewählt aus Gly, D-Glu, D-Asp, D- und L-Cys(SO₃H), F, Cl, Br und I
   ausgewählt ist,
   R₆ aus der Gruppe bestehend aus:
   O, N und S,
   den geschützten oder ungeschützten Aminosäureresten Gly, D-Glu, D-Asp, D- und L-Cys(SO₃H), und Peptidresten mit einer Kettenlänge von 2 bis 200 Aminosäureresten, enthaltend eine Aminosäureart oder eine Kombination von verschiedenen Aminosäurearten ausgewählt aus Gly, D-Glu, D-Asp, D- und L-Cys(SO₃H), ausgewählt ist,
   R₇ aus der Gruppe bestehend aus:
   einer Carboxyl-, Amino-, Thiol-, Hydroxylgruppe,
   F, Cl, Br, I,
   den geschützten oder ungeschützten Aminosäureresten Gly, D-Glu, D-Asp, D- und L-Cys(SO₃H),
   einer Alkyl-, Alkenyl-, Aryl-, Alkylaminocarbonyl-, Arylaminocarbonyl-, Arylalkylaminocarbonyl-, Alkoxycarbonyl-, Aryloxycarbonyl- und Arylalkyloxycarbonylgruppe,
   Peptidresten mit einer Kettenlänge von 2 bis 200 Aminosäureresten, enthaltend eine Aminosäureart oder eine Kombination von verschiedenen Aminosäurearten ausgewählt aus Gly, D-Glu, D-Asp, D- und L-Cys(SO₃H),
   ausgewählt ist, und
   R₈ der Gruppe R₅ wie vorstehend definiert entspricht, wobei R₈ und R₅ gleich oder verschieden voneinander sein können.

Die erfindungsgemäßen Cyclosporinderivate weisen eine pharmazeutische Wirksamkeit auf, beispielsweise bei chronisch entzündlichen Erkrankungen.

Die erfindungsgemäßen Cyclosporinderivate können bei pH 6 eine negative Nettoladung aufweisen, weshalb sie bei entsprechenden Bedingungen eine Zellmembran nicht durchwandern können.

Die Bestimmung der Nettoladung einer Peptidsequenz kann in erster Näherung mittels Berechnung, wie dies z.B. in WO 2003/097706 aufgeführt ist, oder genauer durch entsprechende biochemische Experimente wie z.B. mittels isoelektrischer Fokussierung (vgl. Lexikon der Biochemie, 1. Auflage, 2005, Spektrum Akademischer Verlag) oder durch Titration (vgl. z.B. Fresenius' Journal of Analytical Chemistry 274 (1975), 359-361), Helvetica Chimica Acta 91 (2008), 468-482) ermittelt werden.

Es ist dem Fachmann bekannt, dass sich die Nettoladung eines Moleküls aus der Summe der Teilladungen der einzelnen funktionellen Gruppen ergibt.

Die Erfinder der vorliegenden Anmeldung haben gefunden, dass - um das Verbleiben eines erfindungsgemäßen Cyclosporinderivats im extrazellulären Raum sicher zu gewährleisten - das Cyclosporinderivat ferner keinen Peptidabschnitt umfassen sollte, der in der Lage ist, die Membran einer Zelle zu durchwandern. Unter einem "Peptidabschnitt" wird im Rahmen der Erfindung jeder Abschnitt mit mehr als einer positiv geladenen, d.h. basischen, Aminosäure verstanden, der in der Lage ist, die Membran einer biologischen Zelle zu durchwandern. Die erfindungsgemäßen Cyclosporinderivate sind daher vorzugsweise frei von einem Peptidabschnitt, der in der Lage ist, die Membran einer biologischen Zelle zu durchwandern. Ein Peptidabschnitt bzw. ein Peptid, wie er/es nicht Bestandteil des erfindungsgemäßen Cyclosporinderivats sein darf, kann beispielsweise eine oder mehrere Aminosäuren ausgewählt aus der Gruppe bestehend aus Arginin, Lysin, Histidin oder Kombinationen davon, umfassen. Peptide, die besonders gut in der Lage sind die Membran biologischer Zellen zu durchwandern sind insbesondere Peptide aus 5 bis 20 der vorstehend angeführten Aminosäuren.

In einer bevorzugten Ausführungsform des erfindungsgemäßen Cyclosporinderivats entspricht R₁ einem Rest der Formel III

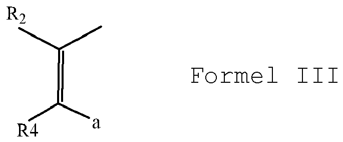

In einer weiteren bevorzugten Ausführungsform des erfindungsgemäßen Cyclosporinderivats entspricht R₁ einem Rest der Formel IV

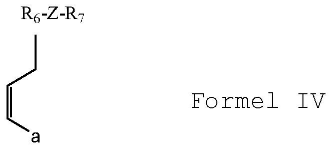

In einer weiteren bevorzugten Ausführungsform des erfindungsgemäßen Cyclosporinderivats entspricht R₁ einem Rest der Formel V

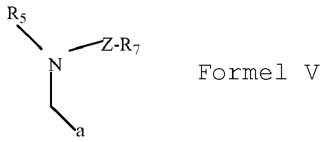

In einer weiteren bevorzugten Ausführungsform des erfindungsgemäßen Cyclosporinderivats entspricht R₁ einem Rest der Formel VI

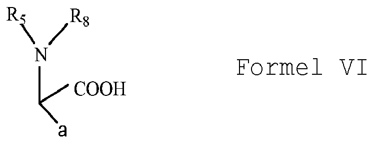

In einer weiteren bevorzugten Ausführungsform des erfindungsgemäßen Cyclosporinderivats entspricht R₁ einem Rest der Formel VII

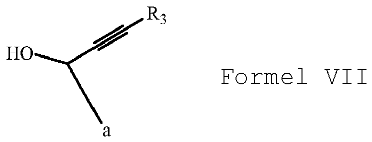

In einer weiteren bevorzugten Ausführungsform des erfindungsgemäßen Cyclosporinderivats entspricht R₁ einem Rest der Formel VIII

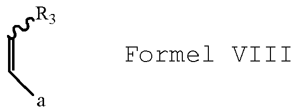

In einer weiteren bevorzugten Ausführungsform des erfindungsgemäßen Cyclosporinderivats entspricht R₁ einem Rest der Formel IX

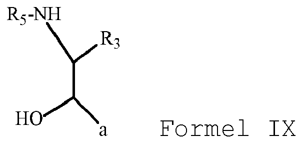

Gemäß einer ganz besonders bevorzugten Ausführungsform der vorliegenden Erfindung ist das Cyclosporinderivat der Formel (I) ausgewählt aus: 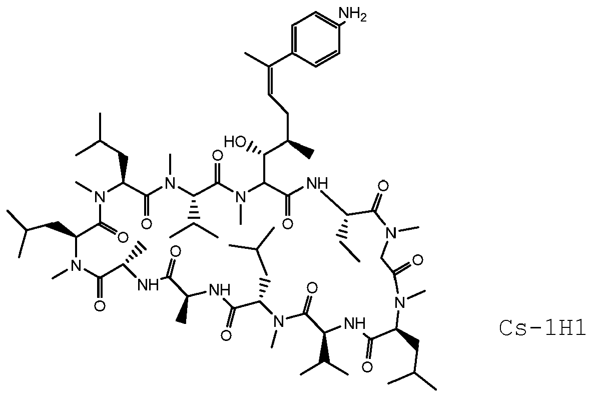 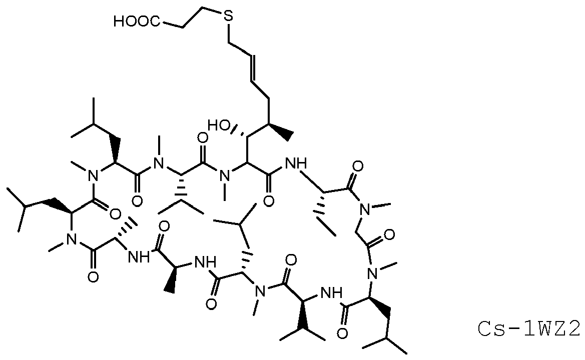 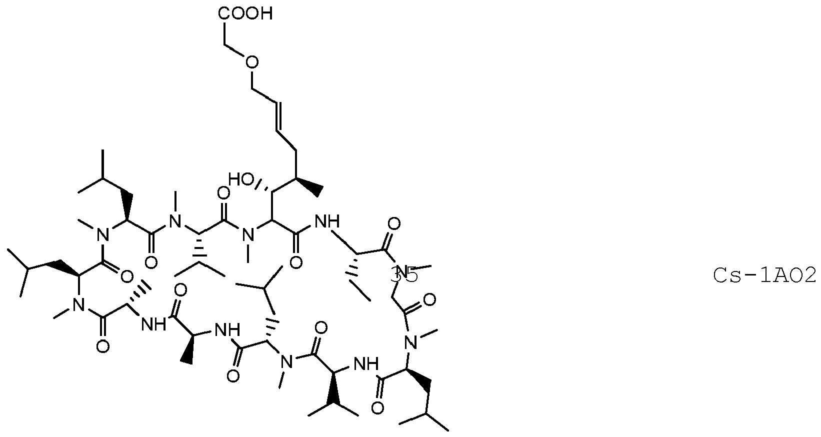 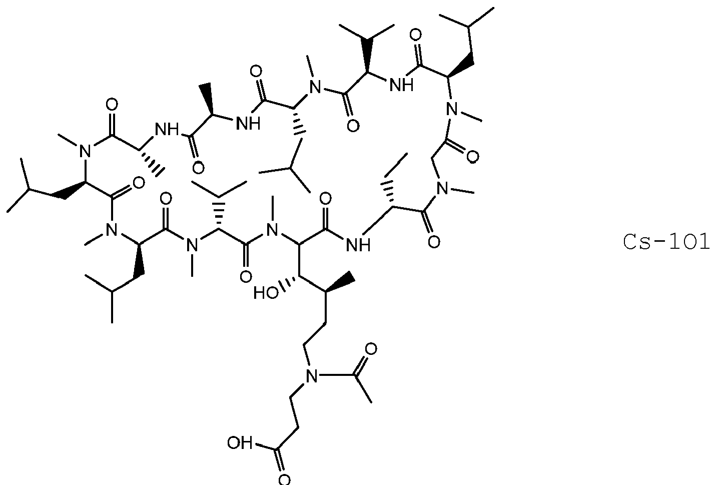 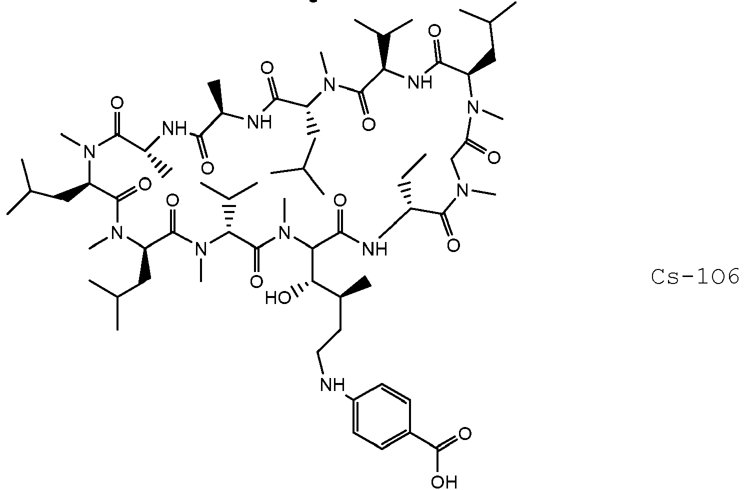 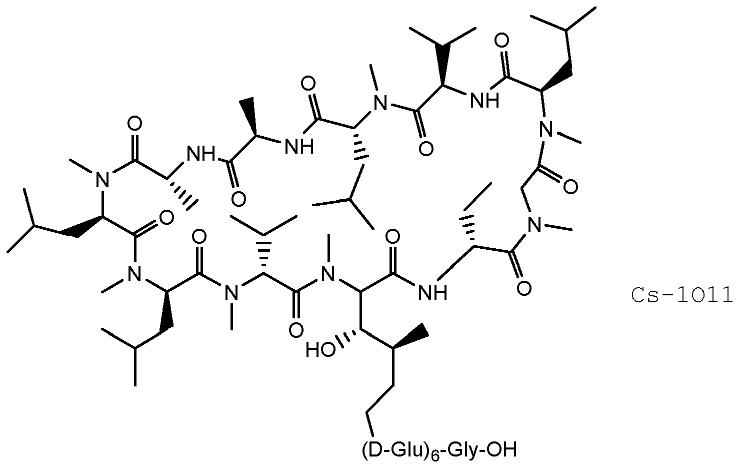 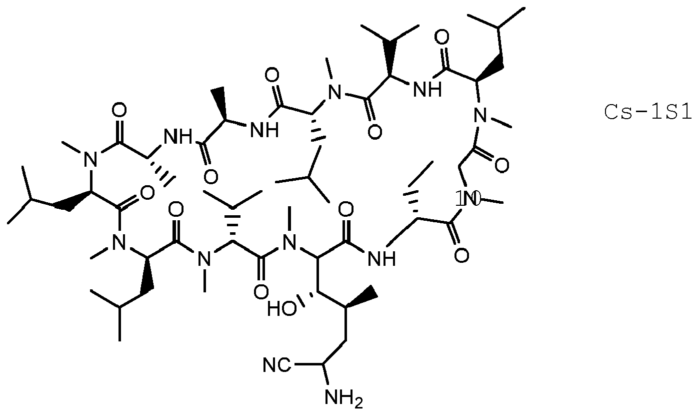 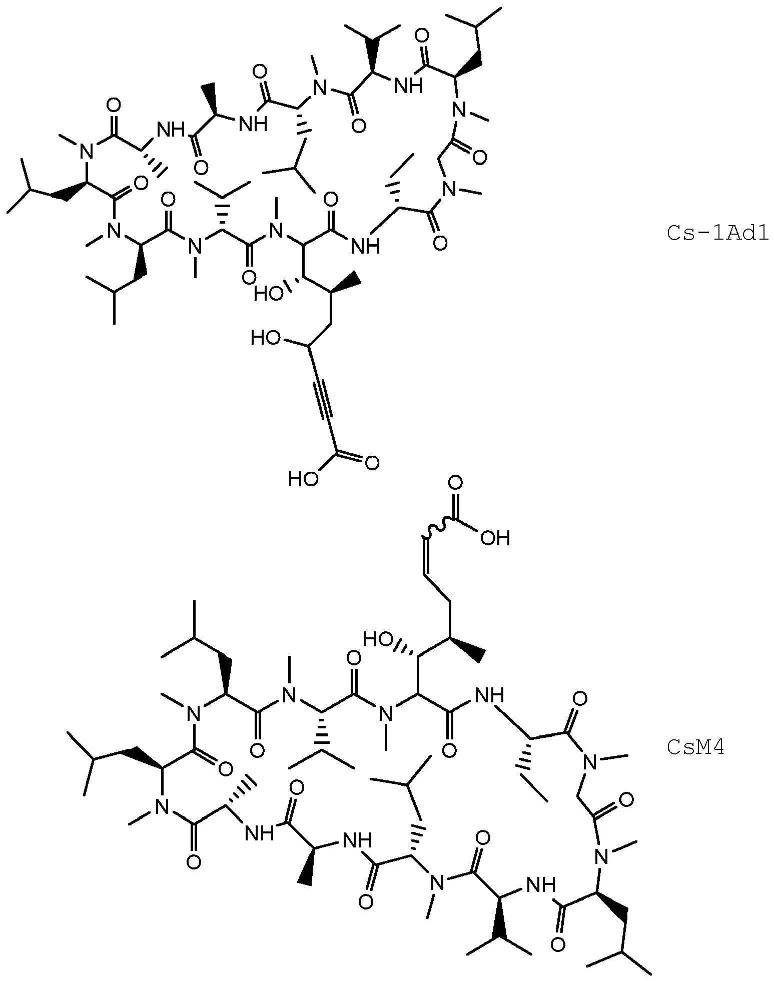 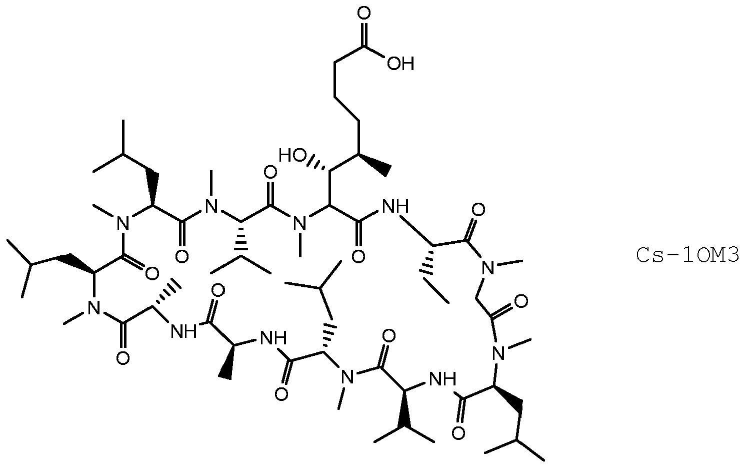 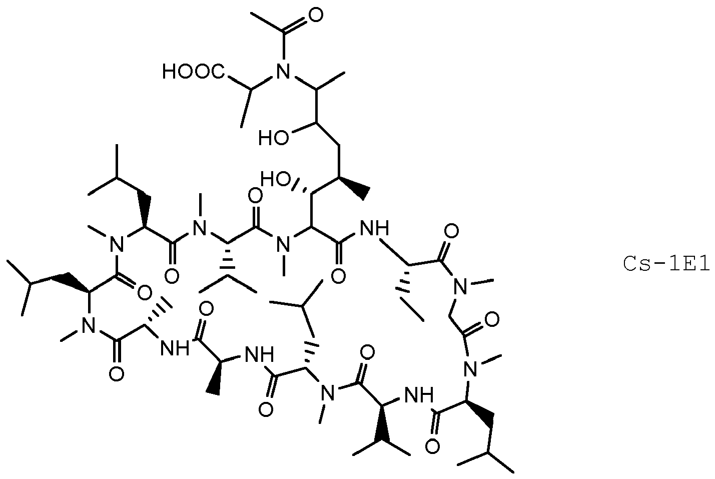 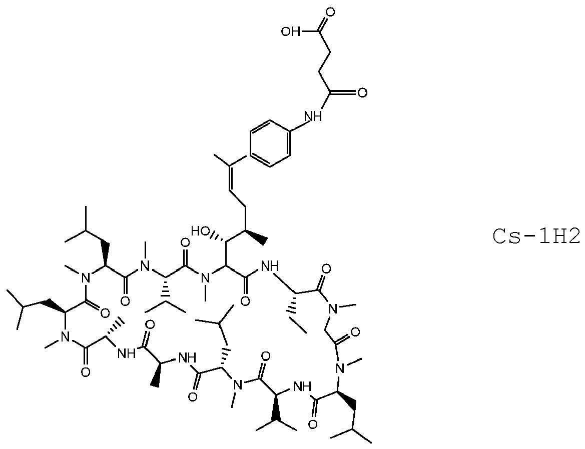 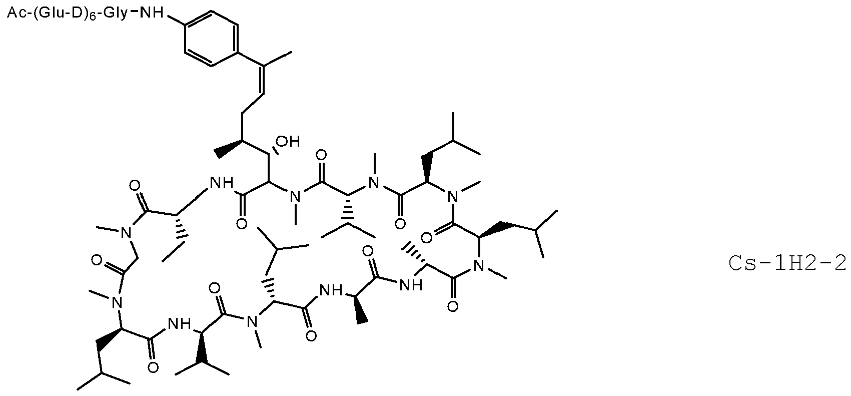 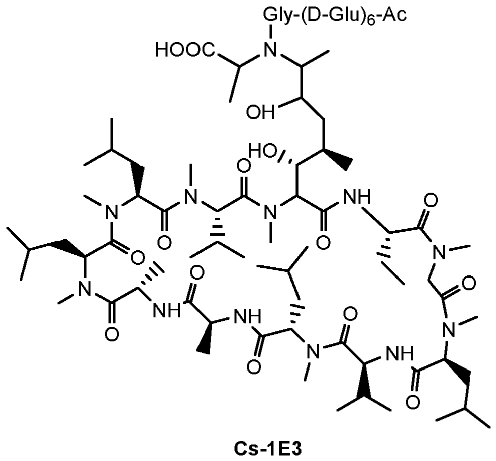 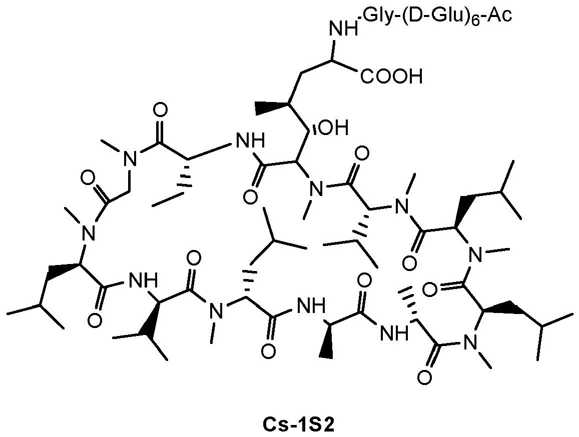 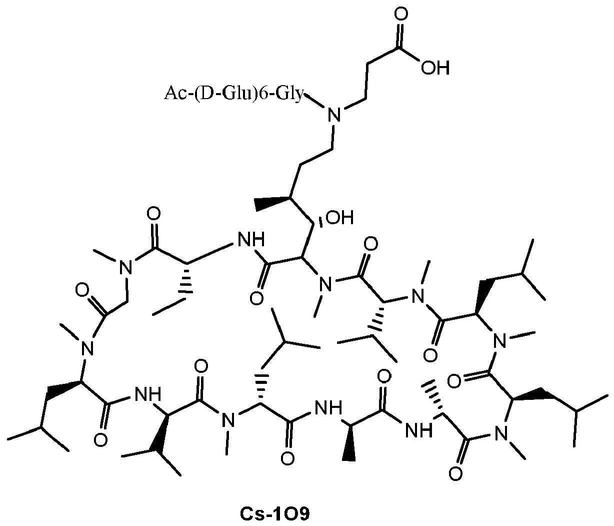 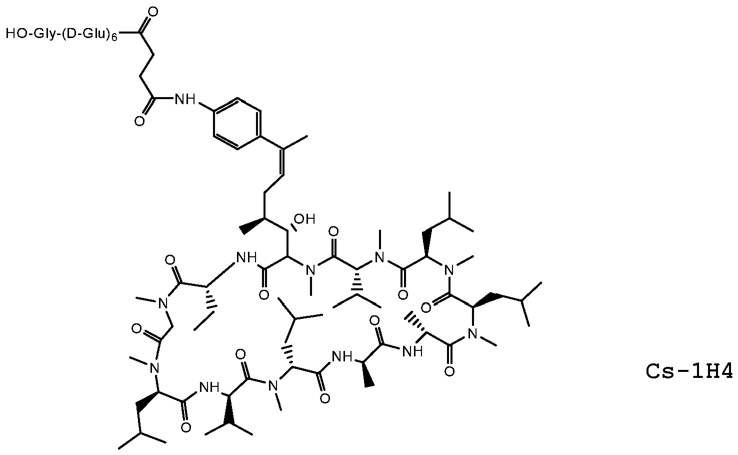 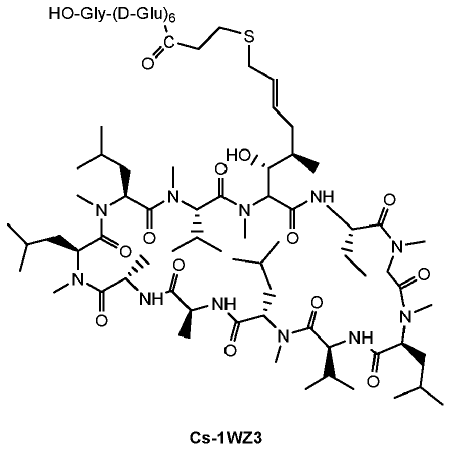 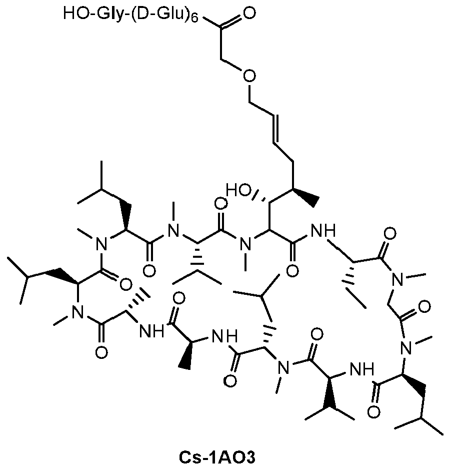 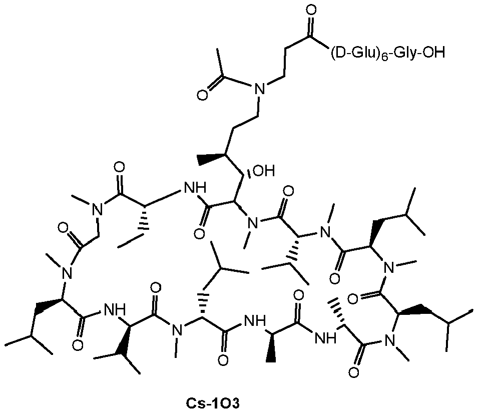 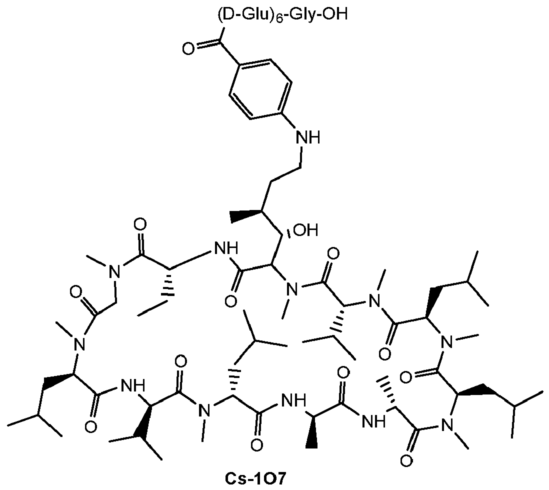 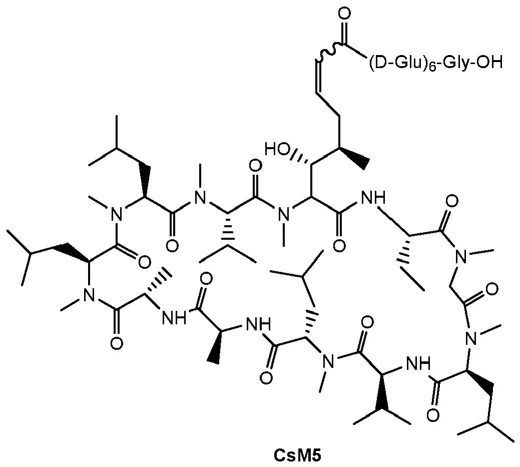 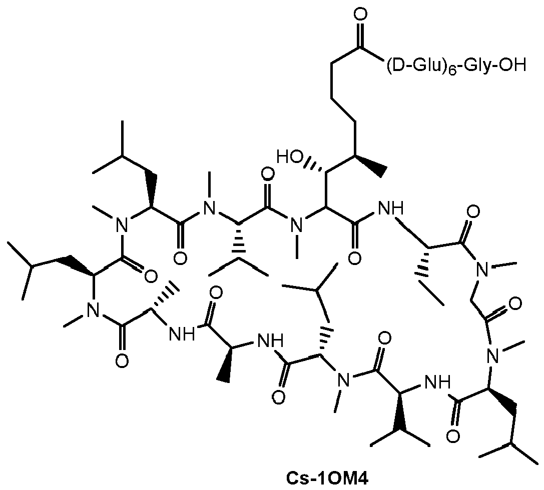 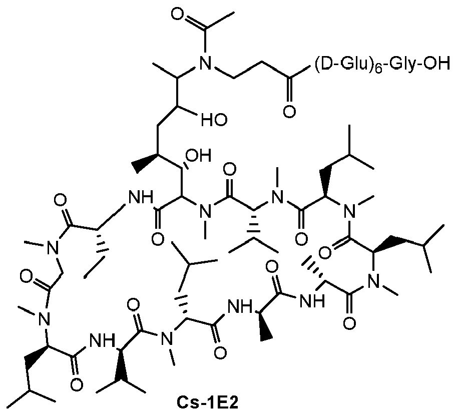 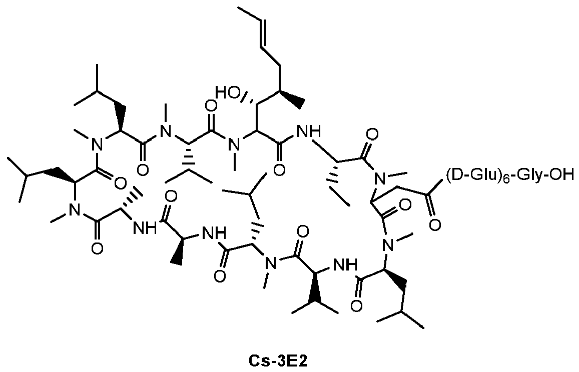 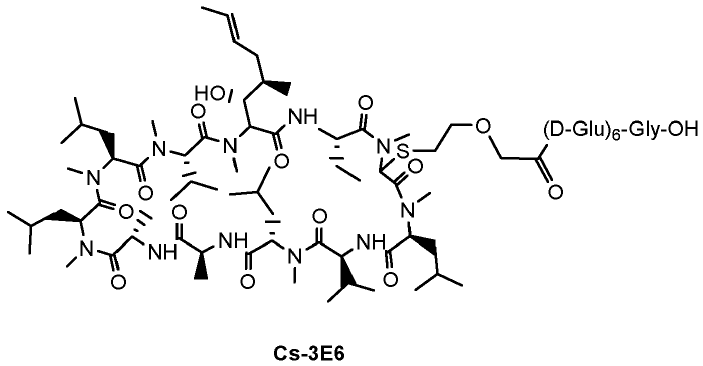 und

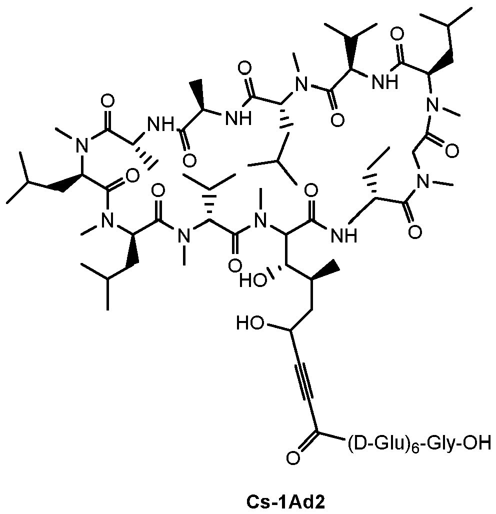

Die vorliegende Erfindung stellt ferner ein Verfahren zur Anreicherung eines Cyclosporinderivats in einem extrazellulären Raum eines multizellulären Objekts bereit, umfassend die Schritte:
- Bereitstellen eines erfindungsgemäßen Cyclosporinderivats;
- Inkontaktbringen des Cyclosporinderivats mit einem multizellulären Objekt.

Unter einem "extrazellulären Raum" sollen alle die Bereiche verstanden werden, welche sich außerhalb des Zytosols und der das Zytosol umschließenden Membran befinden. Dazu gehört auch die beispielsweise in Zellsuspensionen vorhandene Kulturlösung.

Bei dem multizellulären Objekt kann es sich um jedes Objekt handeln, dass aus mindestens zwei gleichen oder unterschiedlichen biologischen Zellen besteht. Der Begriff "biologische Zelle" umfasst dabei sowohl menschliche, tierische als auch pflanzliche und bakterielle Zellen sowie einzellige Lebewesen. Handelt es sich bei den biologischen Zellen um bakterielle Zellen oder einzellige Lebewesen, so wird unter dem Begriff "multizelluläres Objekt" eine Ansammlung mehrerer Zellen, wie beispielsweise eine Zellkolonie einer Bakterienkultur, verstanden. Handelt es sich bei den biologischen Zellen um menschliche oder tierische Zellen, so wird unter dem Begriff "multizelluläres Objekt" ein separiertes Körperteil, wie beispielsweise ein Transplantat, insbesondere ein Organ-, ein Zell-, ein Gliedmaßen- oder ein Gewebetransplantat, Blut oder eine Blutfraktion, wie beispielsweise Blutplasma oder eine in-vitro Kultur menschlicher und/oder tierischer Zellen, wie beispielsweise eine zweidimensionale Gewebekultur oder eine Spheroidkultur der Zellen, verstanden. Handelt es sich bei den biologischen Zellen um Pflanzenzellen, so wird unter dem Begriff "multizelluläres Objekt" ein Teil einer Pflanze, wie beispielsweise Blätter, Wurzel oder Stengel oder auch eine ganze Pflanze verstanden.

Gemäß einer bevorzugten Ausführungsform des erfindungsgemäßen Verfahrens ist das multizelluläre Objekt ein separiertes Organ oder Körperteil, Blut oder eine Blutfraktion, eine Zellkultur oder eine Pflanze.

Die vorliegende Erfindung stellt ferner die Verwendung eines erfindungsgemäßen Cyclosporinderivats als Medikament bereit.

Anwendungsbereiche des erfindungsgemäßen Medikaments können Therapie und Diagnose von Krankheiten aber auch kosmetischer Art sein, wobei unter Therapie im weitesten Sinn auch die Bekämpfung von Schädlingen im Tier- und Pflanzenreich bzw. die Unterstützung von Heilungsprozessen im Tier- und Pflanzenreich aber auch die Beeinflussung biologischer Prozesse in gewünschter Weise verstanden werden soll. Besondere Vorteile liegen dabei in der Tier- und Humanmedizin, insbesondere bei der Applikation von Stoffen auf oder in Zellsuspensionen, Gewebekulturen, Transplantaten oder dem gesamten Säugetiere.

Die vorliegende Erfindung stellt ferner die Verwendung eines erfindungsgemäßen Cyclosporinderivats zur Herstellung eines Medikaments zur Behandlung einer chronischen entzündlichen Erkrankung bereit.

Gemäß einer bevorzugten Ausführungsform der erfindungsgemäßen Verwendung ist die chronische entzündliche Erkrankung Asthma, rheumatoide Arthritis, Multiple Sklerose, Psoriasis oder ulzerative Kolitis.

Die vorliegende Erfindung stellt schließlich ferner eine pharmazeutische Zusammensetzung bereit, die ein erfindungsgemäßes Cyclosporinderivat umfasst.

Bei der erfindungsgemäßen pharmazeutischen Zusammensetzung kann es sich dabei um jedwede pharmazeutische Zusammensetzung handeln, die dem Fachmann als geeignet bekannt ist. Beispielsweise kann das erfindungsgemäße Cyclosporinderivat in einer Form, ausgewählt aus der Gruppe bestehend aus Injektionen, Infusionen, Tabletten, Cremes, Sprays, Kapseln, Sirupen, Emulsionen, Pudern, Pulvern, Zäpfchen oder Ähnlichen eingesetzt werden.

In einer bevorzugten Ausführungsform handelt es sich bei der pharmazeutischen Zusammensetzung um Sprays oder Tabletten.

Im Rahmen der vorliegenden Erfindung kann das Cyclosporinderivat ferner Gruppen umfassen, die das Cyclosporinderivat mit weiteren Eigenschaften versehen, die für den Fachmann für den jeweiligen Einsatzzweck wünschenswert sind. Dabei kann das erfindungsgemäße Cyclosporinderivat mit einer, aber auch mit mehreren Gruppen verbunden werden, die entweder gleich, gleichartig oder unterschiedlich sein können. Die Aufnahme zusätzlicher Gruppen in das erfindungsgemäße Cyclosporinderivat kann dabei zum Einen dazu dienen, bereits vorhandene Eigenschaften zu verstärken, zum Anderen ist es aber auch möglich das Cyclosporinderivat mit neuen, weiteren Eigenschaften zu versehen. Es ist beispielsweise denkbar, dass das Cyclosporinderivat mit einem Indikator versehen wird, um dessen Anreicherung im gewünschten Gewebe zu kontrollieren oder um das gewünschte Gewebe mittels Indikatorverteilung klassifizieren zu können. Weiter ist es denkbar, dass das Cyclosporinderivat mit einer Gruppe versehen wird, die seine Anreicherung in ganz bestimmten Geweben ermöglicht.

In einer bevorzugten Ausführungsform umfasst das Cyclosporinderivat daher einen Indikator, der insbesondere kovalent an das Cyclosporinderivat gebunden sein kann. Der Indikator kann jedoch grundsätzlich mit dem Cyclosporinderivat auf jedwede Art verbunden werden, die dem Fachmann als für den erfindungsgemäßen Zweck geeignet bekannt ist.

Unter dem Begriff "Indikator" sind im Sinne der Erfindung Stoffe wie z.B. Farbstoffe, spannungssensitive Indikatoren, pH-Wert-sensitive Indikatoren, Calcium-sensitive Indikatoren, radioaktive Elemente, NMR-Label oder aber auch Elektronenspinlabel zusammengefasst und in der wissenschaftlichen Literatur mehrfach beschrieben (WO/2005/022158, EP 0649022, US 6,596,499, US 7,090,995, US 4,672,044). Der Begriff Indikator umfasst im Sinne der Erfindung einzelne Atome oder Moleküle, welche kovalent mit dem Cyclosporinderivat verbunden sind. Dabei kann ein Indikator oder auch mehrere Indikatoren direkt an das Cyclosporinderivat kovalent gebunden sein. Bevorzugt umfasst der Begriff "Indikator" Farbstoffe, spannungssensitive Indikatoren, pH-Wert-sensitive Indikatoren, radioaktive Elemente, Calcium-sensitive Indikatoren, NMR-Label und Elektrospinlabel.

"Farbstoffe" im Sinne der Erfindung sind Stoffe, die optisch durch Detektion der von ihnen ausgesandten oder der durch sie nicht absorbierten elektromagnetischen Strahlung nachgewiesen werden können. Dazu gehören z.B. Farbstoffe wie Fluoresceinisocyanat (FIC), Fluoresceinisothiocyanat (FITC), Dimethylaminonaphthalin-S-sulfonylchlorid (DANSC), Tetramethylrhodaminisothiocyanat (TRITC), Lissaminrhodamin-B200-sulfonylchlorid (RB 200 SC), usw. Eine Beschreibung zahlreicher geeigneter Moleküle ist z.B. bei DeLuca, "Immunofluorescence Analysis", in "Antibody As A Tool", Marchalonis et al., Hrsg., John Wiley & Sons, Ltd., Seiten 189-231, (1985) zu finden.

"Spannungssensitive Indikatoren" im Sinne der Erfindung sind Stoffe, die in Abhängigkeit einer anliegenden elektrischen Potentialdifferenz oder des vorliegenden elektrischen Potentials ihre physikalischen, optischen oder katalytischen Eigenschaften derart ändern, dass diese ein detektierbares Signal hervorrufen. Dem Fachmann sind spannungssensitive Indikatoren wie z.B. DIBAC bekannt (Japanese Journal of Pharmacology 86 (2001), 342-350, American Journal of Physiology - Heart & Circulatory Physiology 287 (2004), H985-H993).

"pH-Wert-sensitive Indikatoren" im Sinne der Erfindung sind Stoffe, die in Abhängigkeit des pH-Wertes derart ihre physikalischen, optischen oder katalytischen Eigenschaften ändern, dass diese ein detektierbares Signal hervorrufen. Solche Indikatorfarbstoffe, wie z.B. Phenolrot, Bromthymolblau, Bromphenolblau, usw., sind dem Fachmann bekannt.

"Calcium-sensitive Indikatoren" im Sinne der Erfindung sind Stoffe, die in Anwesenheit von Calcium derart ihre physikalischen, optischen oder katalytischen Eigenschaften ändern, dass diese ein detektierbares Signal hervorrufen. Dem Fachmann bekannte Calcium-sensitive Indikatoren sind z.B. Aequorin und andere Calcium-sensitive Farbstoffe, wie z.B. FURA-2.

"Radioaktive Elemente" im Sinne der Erfindung erzeugen z.B. Gammastrahlung, wie z.B. folgende Isotope ¹²⁴J, ¹²⁵J, ¹²⁸J, ¹³¹J, ¹³²J oder ⁵¹Cr, wobei ¹²⁵J besonders bevorzugt ist. Andere, wie z.B. ¹¹C, ¹⁸F_{,} ¹⁵O oder ¹³N, können mittels ihrer Positronenstrahlung und entsprechender Detektoren (Positronen-Emissions-Tomographie) nachgewiesen werden, und andere, wie z.B. ¹¹¹In, lassen sich mittels Elektroneneinfang ("electron capture") nachweisen.

"NMR-Label" im Sinne der Erfindung sind Substanzen, in denen Atome mit ungerader Nukleonenanzahl (Summe der Protonen und Neutronen) enthalten sind. Solche Atomkerne, wie z.B. ¹³C, ¹⁵N oder ¹⁹F, besitzen einen Kernspin und damit ein kernmagnetisches Moment.

"Elektronenspinlabel" dienen im Sinne der Erfindung zur Messung der "Electron Paramagnetic Resonance" mittels Elektronenspinresonanz. Dabei wird die resonante Mikrowellenabsorption einer Probe in einem äußeren Magnetfeld gemessen. Damit lassen sich Moleküle nachweisen, die über ein permanentes magnetisches Moment (ungepaarte Elektronen) verfügen (Physics in Medicine & Biology 43 (1998), U 3-U 4, Clinical Chemistry & Laboratory Medicine 46 (2008), 1203-1210).

Das erfindungsgemäße Cyclosporinderivat kann dabei einen oder auch mehrere Indikator(en) enthalten, die gleicher oder aber auch unterschiedlicher Natur sein können.

Die Verwendung von Indikatoren ist besonders vorteilhaft, wenn das erfindungsgemäße Cyclosporinderivat zur Herstellung eines Medikaments zur Anwendung in einem therapeutischen Verfahren, wie beispielsweise einem Diagnoseverfahren, eingesetzt werden soll (z.B. Anamneseerhebung, körperliche Untersuchung, Anwendung bildgebender Verfahren wie Röntgen/MRT oder Analytik mit Laborwerten des Bluts und anderen Körperflüssigkeiten). Wenn das erfindungsgemäße Cyclosporinderivat noch einen oder mehrere Indikator(en) enthält, kann der Verteilungsraum des Cyclosporinderivats anhand dieser Indikatoren erkannt werden. Indikatoren können überdies genutzt werden um das Cyclosporinderivat zu quantifizieren.

In einer weiteren bevorzugten Ausführungsform ist das erfindungsgemäße Cyclosporinderivat zudem frei von einer Protease-Schnittstelle, wie einem Linker aus Aminosäuren, der beispielsweise der Verbindung der einzelnen Gruppen dienen kann. Werden einzelne Gruppen nicht direkt, sondern über einen spaltbaren Linker verbunden, so könnte es unter Umständen - beispielsweise eine unbeabsichtigte Nebenwirkung bei der Verabreichung mehrerer Medikamente - zu einer unbeabsichtigten Spaltung und somit zum Verlust der jeweils über den Linker verknüpften Gruppe kommen.

Die vorliegende Erfindung betrifft zudem die folgenden Ausführungsformen:

Ausführungsform (i): Cyclosporinderivat der allgemeinen Formel (I) 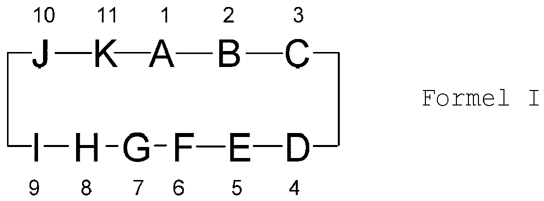 oder ein pharmazeutisch verträgliches Salz davon,
wobei in Formel (I)
A ein Aminosäurerest der Formel (II) ist, 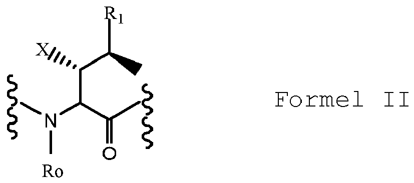 bei dem R₁ einem Rest der Formel III 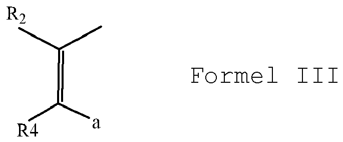 entspricht, der über das Kohlenstoffatom a kovalent verknüpft ist, oder
   bei dem R₁ einem Rest der Formel IV 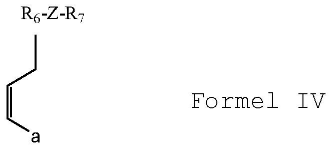 entspricht, der über das Kohlenstoffatom a kovalent verknüpft ist, oder
   bei dem R₁ einem Rest der Formel V 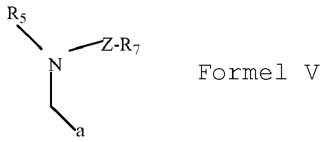 entspricht, der über das Kohlenstoffatom a kovalent verknüpft ist, oder
   bei dem R₁ einem Rest der Formel VI 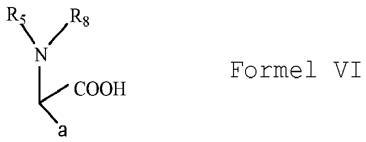 entspricht, der über das Kohlenstoffatom a kovalent verknüpft ist, oder
   bei dem R₁ einem Rest der Formel VII 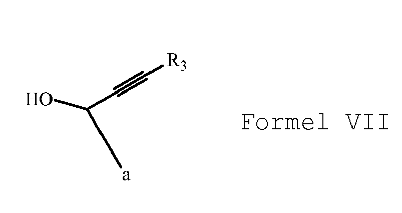 entspricht, der über das Kohlenstoffatom a kovalent verknüpft ist, oder
   bei dem R₁ einem Rest der Formel VIII 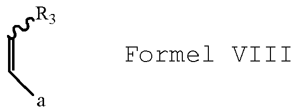 entspricht, der über das Kohlenstoffatom a kovalent verknüpft ist, oder bei dem R₁ einem Rest der Formel IX entspricht 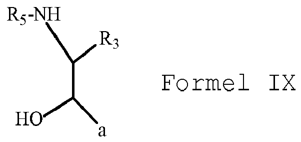 der über das Kohlenstoffatom a kovalent verknüpft ist,
   wobei die Gruppe C=O in der Formel II über eine Amidbindung kovalent an einer alpha-Aminogruppe von B gebunden ist und die Gruppe N-Rₒ in der Formel II über eine Amidbindung kovalent an einer Carboxylgruppe von K gebunden ist,
B ein Aminosäurerest ist, der aus der Gruppe bestehend aus:
   alpha-Aminobuttersäure,
   Alanin,
   Threonin,
   Valin,
   Norvalin und
   in der Seitenkette mit einer Hydroxylgruppe
   modifizierter alpha-Aminobuttersäure, Alanin,
   Threonin, Valin oder Norvalin
   ausgewählt ist,
C ein Sarkosinrest ist,
D ein Aminosäurerest ist, der aus der Gruppe bestehend aus:
   Leucin,
   N-Methylleucin,
   Valin,
   gamma-Hydroxy-N-methylleucin und
   gamma-Hydroxyleucin
   ausgewählt ist,
E ein Aminosäurerest ist, der aus der Gruppe bestehend aus:
   Valin,
   Norvalin und
   einem modifizierten Valin oder Norvalin, bei dem ein Kohlenstoffatom der Seitenkette mit einer
   Hydroxylgruppe substituiert ist,
   ausgewählt ist,
F ein Aminosäurerest ist, der aus der Gruppe bestehend aus:
   Leucin,
   N-Methylleucin,
   gamma-Hydroxy-N-methylleucin und
   gamma-Hydroxyleucin
   ausgewählt ist,
G ein alpha-Aminobuttersäurerest oder ein Alaninrest ist,
H ein D-Alaninrest ist,
I und J Reste sind, die unabhängig voneinander aus der Gruppe bestehend aus:
   Leucin,
   N-Methylleucin,
   gamma-Hydroxy-N-methylleucin und
   gamma-Hydroxyleucin
   ausgewählt sind, und
K ein N-Methylvalinrest oder ein Valinrest ist,
   wobei in den Formeln II bis IX
   Rₒ für H oder CH₃ steht,
   X für H oder
   Hydroxyl oder
   eine Hydroxylgruppe steht, die mit einer Alkanoyl-, Aryloyl-, Alkylaminocarbonyl-, Arylaminocarbonyl-, Arylalkylaminocarbonyl-, Alkoxycarbonyl-, Aryloxycarbonyl-, Arylalkyloxycarbonylgruppe oder einer Silylschutzgruppe derivatisiert ist,
   R₂ für H oder
   einen substituierten oder unsubstituierten monozyklischen, bizyklischen oder trizyklischen Aryl- oder Heteroarylring, der gegebenenfalls mit R₃ substituiert ist, steht,
   R₃ aus der Gruppe bestehend aus:
   - Z-NY-Y₁,
   - Z-CO-Y,
   - Z-S-Y,
   - Y₂,
   - NY-Y₁,
   - CO-Y,
   - S-Y,
   - Z-O-Y und
   - O-Y
   ausgewählt ist,
   R₄ der Gruppe R₂ wie vorstehend definiert entspricht, wobei R₄ und R₂ gleich oder verschieden voneinander sein können,
   Z aus der Gruppe bestehend aus:
   einer unverzweigten, verzweigten oder zyklischen Alkylgruppe mit 1 bis 20 Kohlenstoffatomen,
   einer unverzweigten, verzweigten oder zyklischen Alkenylgruppe mit 1 bis 20 Kohlenstoffatomen, einer unverzweigten, verzweigten oder zyklischen Alkinylgruppe mit 1 bis 20 Kohlenstoffatomen, einem substituierten oder unsubstituierten monozyklischen, bizyklischen oder trizyklischen Aryl- oder Heteroarylring und
   einer Gruppe der allgemeinen Formel -[CH₂)ₘ-O-(CH₂)ₙ]ₒ, wobei m, n und O unabhängig voneinander ganze Zahlen von 1 bis 20 sind,
   ausgewählt ist,
   Y aus der Gruppe bestehend aus:
   H,
   den geschützten oder ungeschützten Aminosäureresten Gly, D-Glu, D-Asp, D- und L-Cys(SO₃H), ungeschützten Peptidresten mit einer Kettenlänge von 2 bis 200 Aminosäureresten, enthaltend eine
   Aminosäureart oder eine Kombination von verschiedenen Aminosäurearten ausgewählt aus Gly, D-Glu, D-Asp, D- und L-Cys(SO₃H), einer Alkyl-, Alkenyl-, Aryl-, Alkylaminocarbonyl-, Arylaminocarbonyl-, Arylalkylaminocarbonyl-, Alkylcarboxyl-, Alkoxycarbonyl-, Aryloxycarbonyl- und Arylalkyloxycarbonylgruppe ausgewählt ist,
   Y₁ der Gruppe Y wie vorstehend definiert entspricht, wobei Y und Y₁ gleich oder verschieden voneinander sein können,
   Y₂ für H, F, Cl, Br oder I steht,
   R₅ aus der Gruppe bestehend aus:
   einer Alkyl-, Alkenyl-, Aryl-, Alkylaminocarbonyl-, Arylaminocarbonyl-, Arylalkylaminocarbonyl-, Alkylcarbonyl-, Alkoxycarbonyl-, Aryloxycarbonyl-, Arylalkyloxycarbonylgruppe,
   den Aminosäureresten Gly, D-Glu, D-Asp, D- und L-Cys(SO₃H),
   Peptidresten mit einer Kettenlänge von 2 bis 200 Aminosäureresten, enthaltend eine Aminosäureart oder eine Kombination von verschiedenen Aminosäurearten, ausgewählt aus Gly, D-Glu, D-Asp, D- und L-Cys(SO₃H), F, Cl, Br und I
   ausgewählt ist,
   R₆ aus der Gruppe bestehend aus:
   O, N und S,
   den geschützten oder ungeschützten Aminosäureresten Gly, D-Glu, D-Asp, D- und L-Cys(SO₃H), und Peptidresten mit einer Kettenlänge von 2 bis 200 Aminosäureresten, enthaltend eine Aminosäureart oder eine Kombination von verschiedenen Aminosäurearten ausgewählt aus Gly, D-Glu, D-Asp, D- und L-Cys(SO₃H),
   ausgewählt ist,
   R₇ aus der Gruppe bestehend aus:
   einer Carboxyl-, Amino-, Thiol-, Hydroxylgruppe, F, Cl, Br, I,
   den geschützten oder ungeschützten Aminosäureresten Gly, D-Glu, D-Asp, D- und L-Cys(SO₃H),
   einer Alkyl-, Alkenyl-, Aryl-, Alkylaminocarbonyl-, Arylaminocarbonyl-, Arylalkylaminocarbonyl-, Alkoxycarbonyl-, Aryloxycarbonyl- und Arylalkyloxycarbonylgruppe,
   Peptidresten mit einer Kettenlänge von 2 bis 200 Aminosäureresten, enthaltend eine Aminosäureart oder eine Kombination von verschiedenen Aminosäurearten ausgewählt aus Gly, D-Glu, D-Asp, D- und L-Cys(SO₃H),
   ausgewählt ist, und
   R₈ der Gruppe R₅ wie vorstehend definiert entspricht, wobei R₈ und R₅ gleich oder verschieden voneinander sein können.

Ausführungsform (ii): Cyclosporinderivat nach Ausführungsform (i), dadurch gekennzeichnet, dass R₁ einem Rest der Formel III 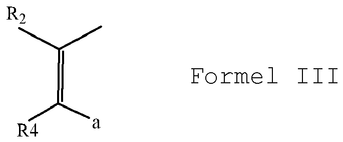 entspricht, der über das Kohlenstoffatom a kovalent verknüpft ist.

Ausführungsform (iii): Cyclosporinderivat nach Ausführungsform (i), dadurch gekennzeichnet, dass R₁ einem Rest der Formel IV 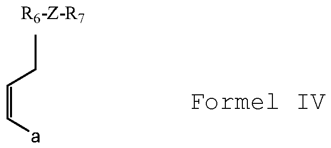 entspricht, der über das Kohlenstoffatom a kovalent verknüpft ist.

Ausführungsform (iv): Cyclosporinderivat nach Ausf+ührungsform (i), dadurch gekennzeichnet, dass R₁ einem Rest der Formel V 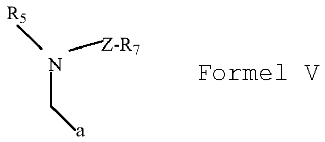 entspricht, der über das Kohlenstoffatom a kovalent verknüpft ist.

Ausführungsform (v): Cyclosporinderivat nach Ausführungsform (i), dadurch gekennzeichnet, dass R₁ einem Rest der Formel VI 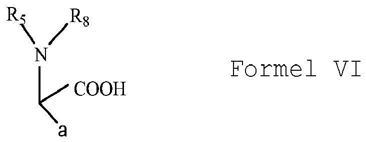 entspricht, der über das Kohlenstoffatom a kovalent verknüpft ist.

Ausführungsform (vi): Cyclosporinderivat nach Ausführungsform (i), dadurch gekennzeichnet, dass R₁ einem Rest der Formel VII 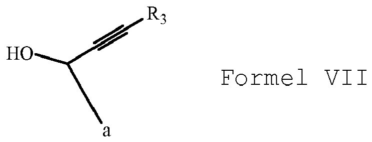 entspricht, der über das Kohlenstoffatom a kovalent verknüpft ist.

Ausführungsform (vii): Cyclosporinderivat nach Ausführungsform (i), dadurch gekennzeichnet, dass R₁ einem Rest der Formel VIII 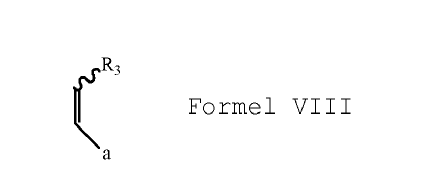 entspricht, der über das Kohlenstoffatom a kovalent verknüpft ist.

Ausführungsform (viii): Cyclosporinderivat nach Ausführungsform (i), dadurch gekennzeichnet, dass R₁ einem Rest der Formel IX entspricht 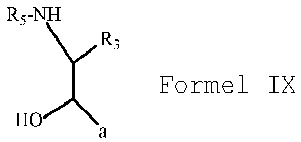 der über das Kohlenstoffatom a kovalent verknüpft ist.

Ausführungsform (ix): Cyclosporinderivat nach Ausführungsform (i), wobei das Cyclosporinderivat ausgewählt ist aus:

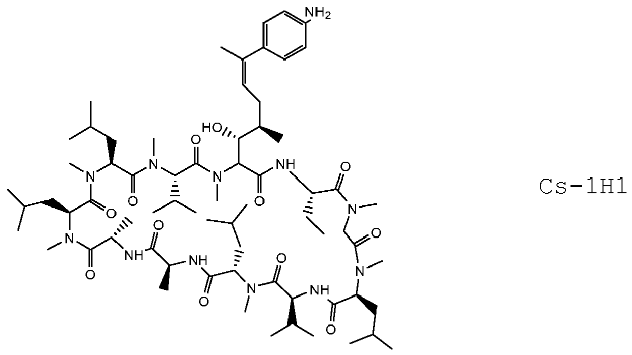

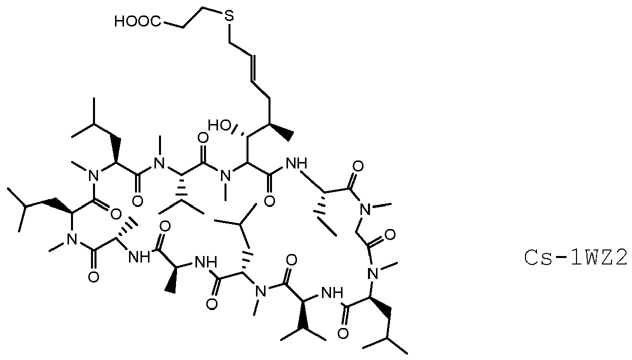

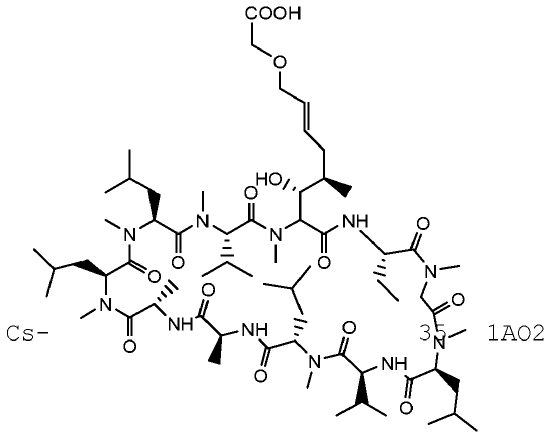

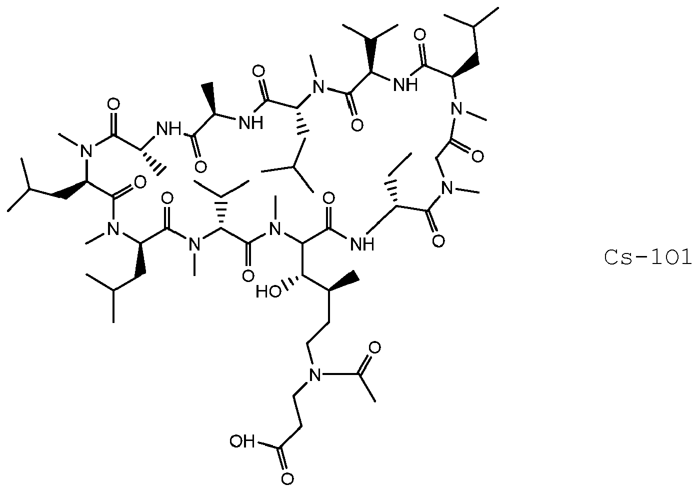

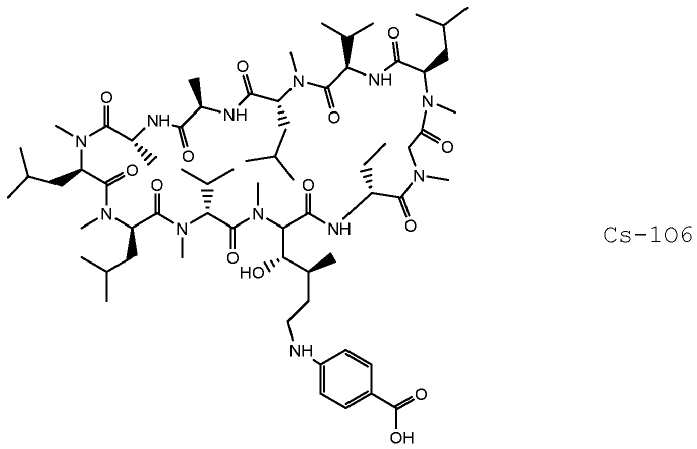

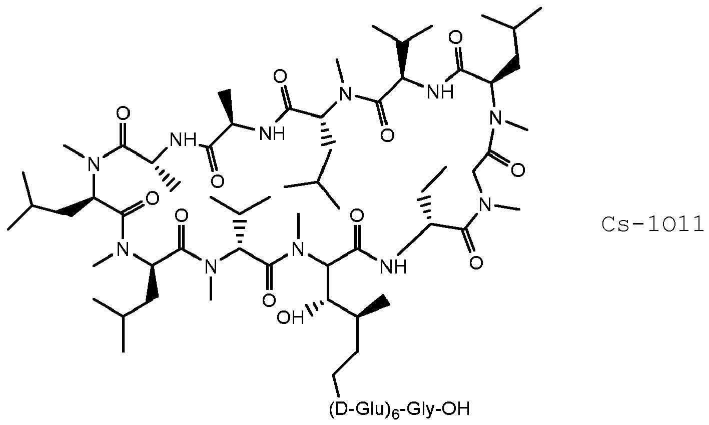

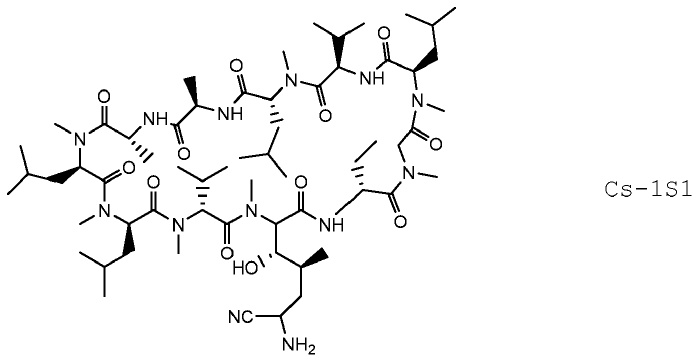

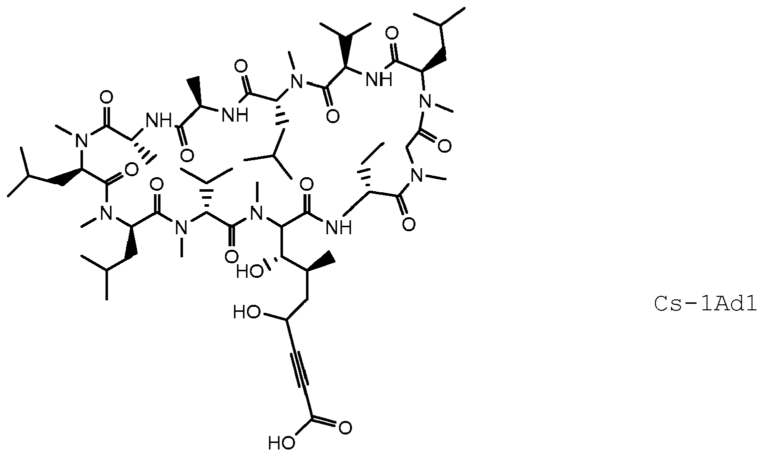

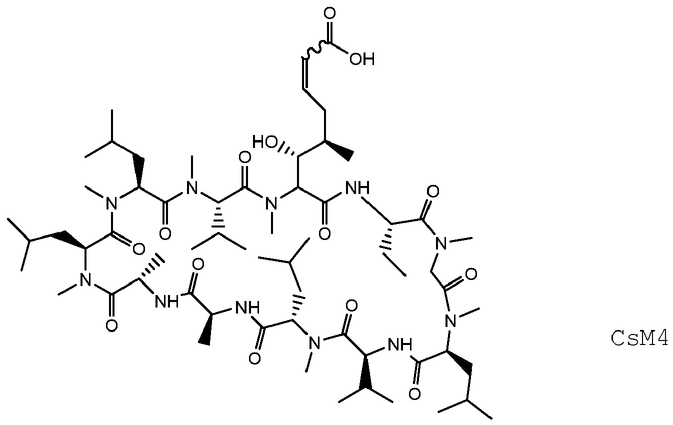

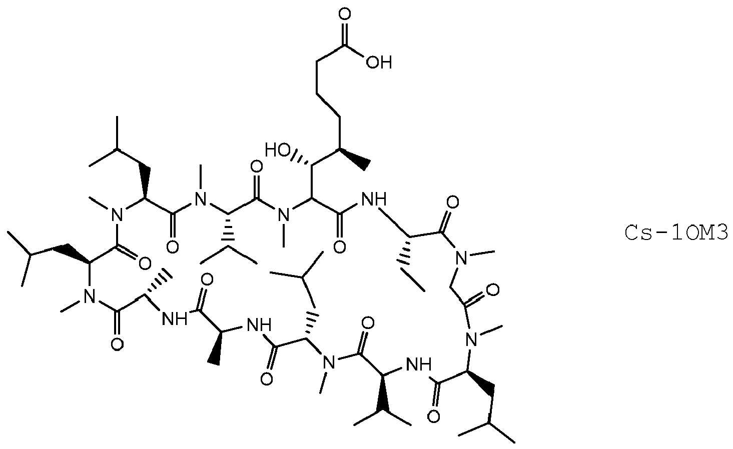

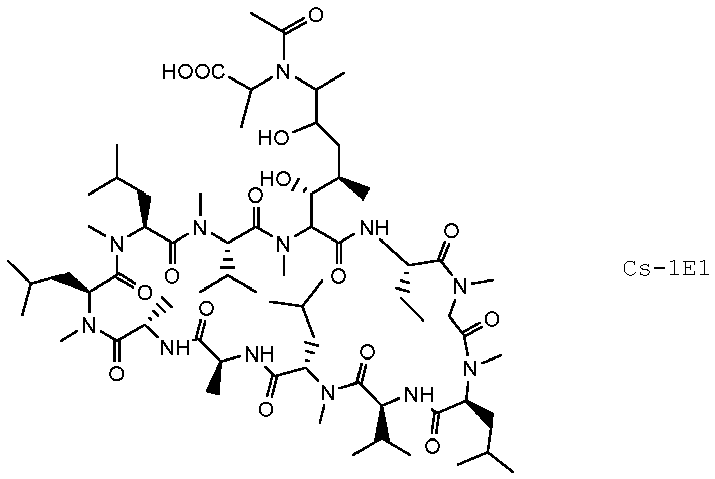

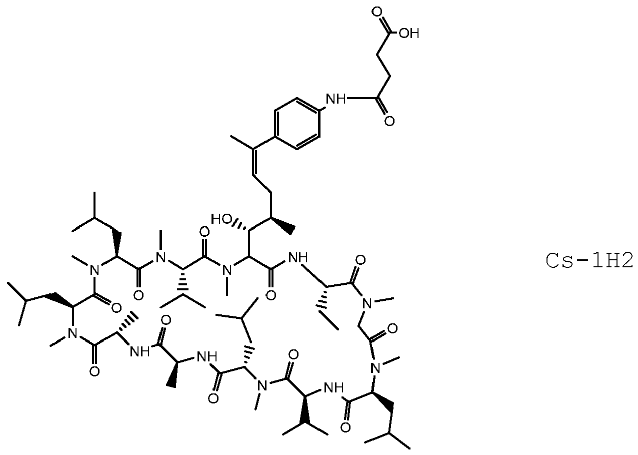

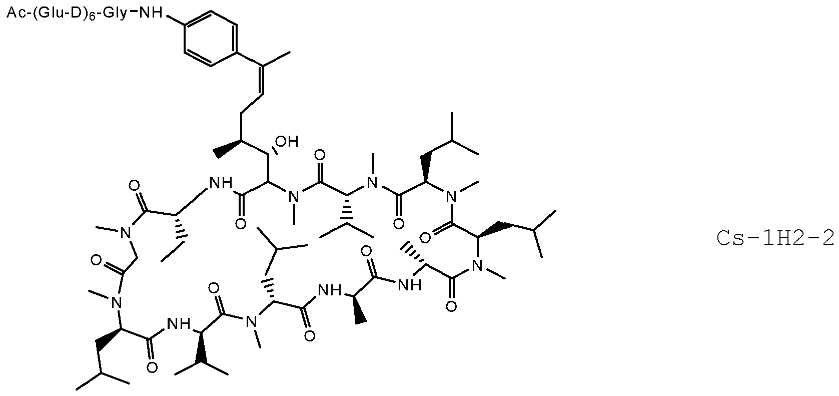

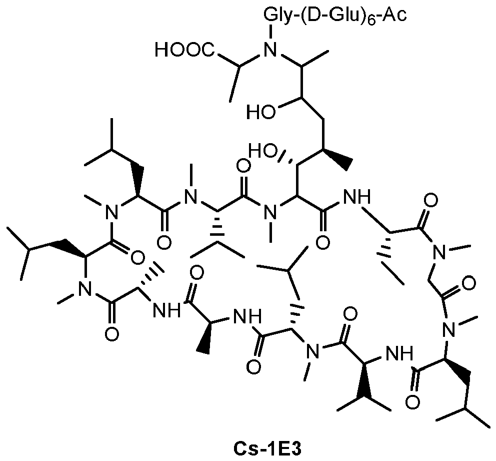

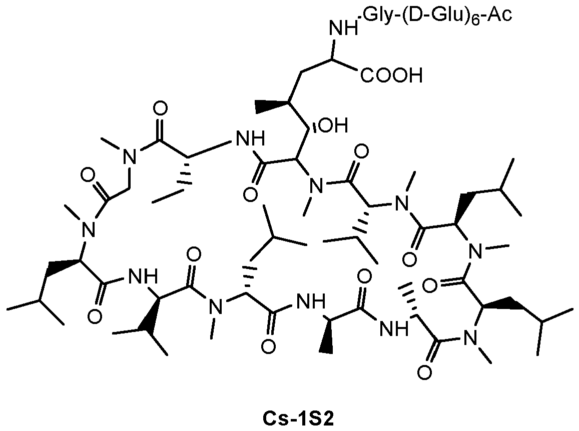

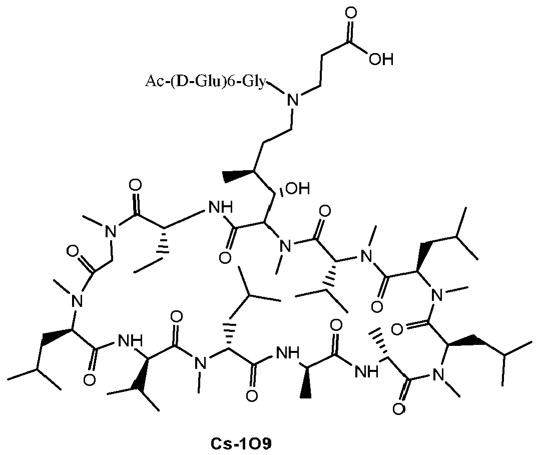

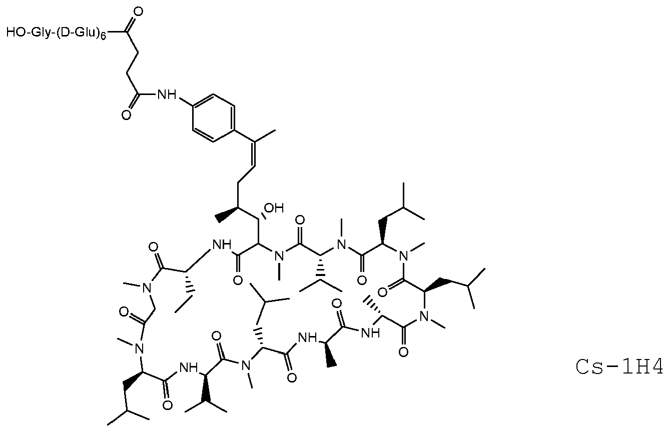

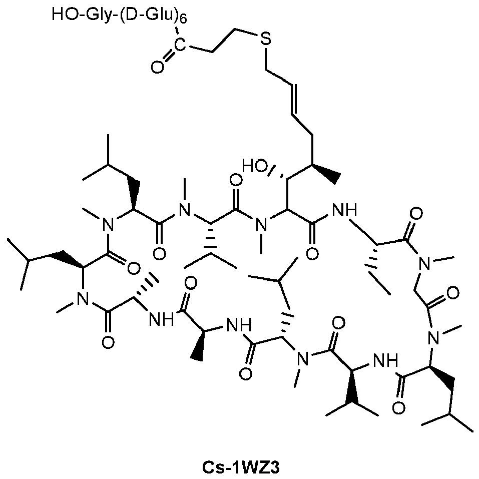

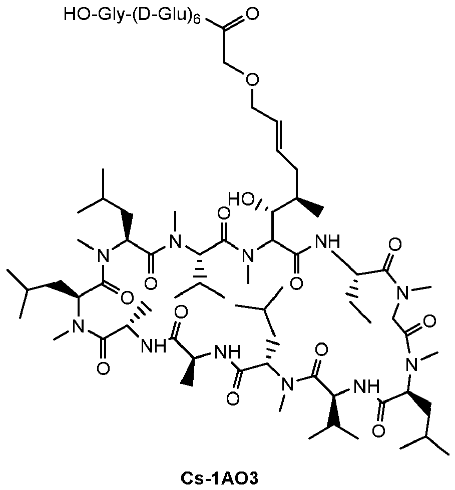

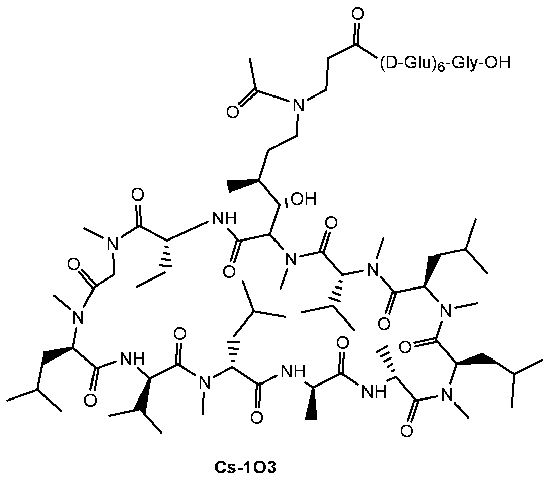

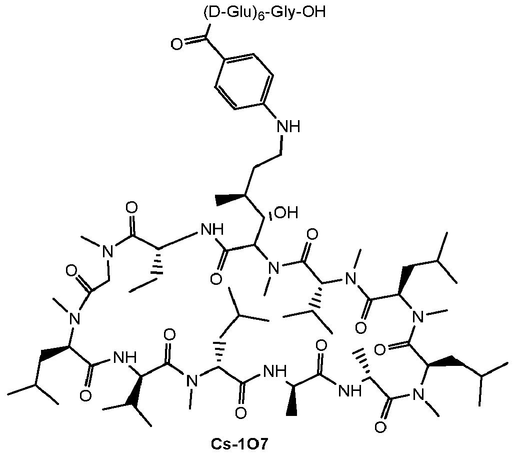

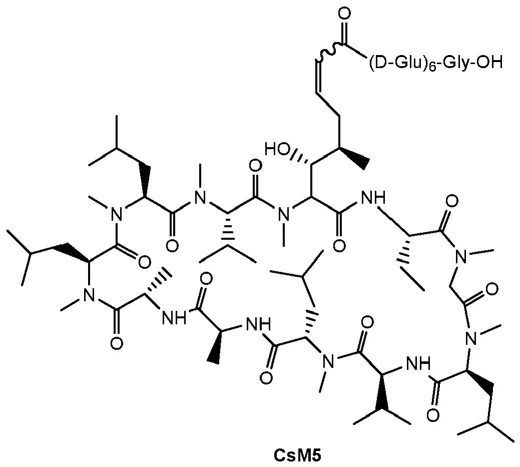

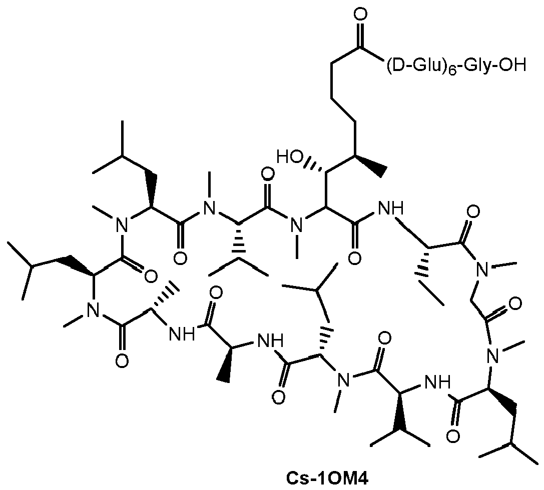

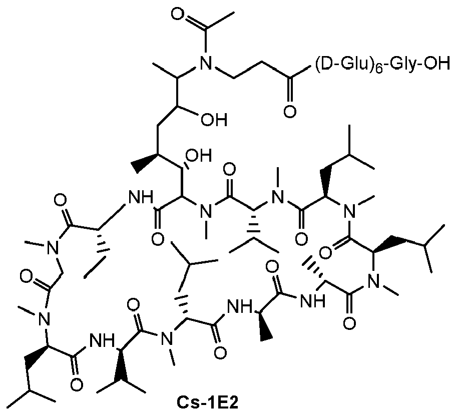

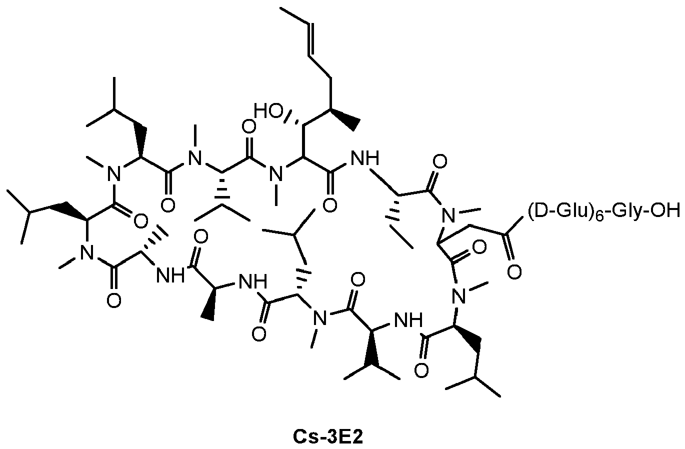

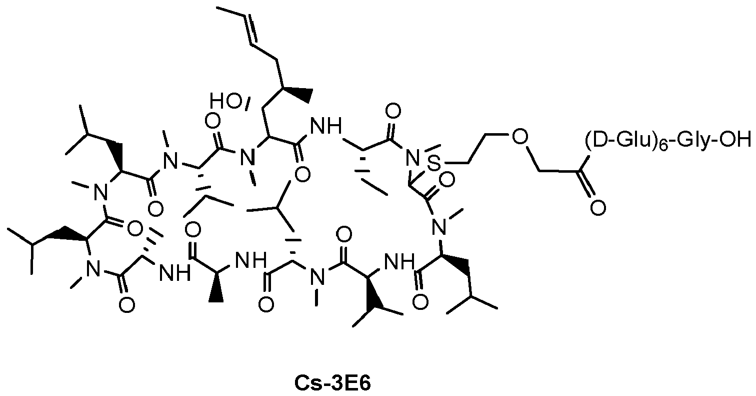

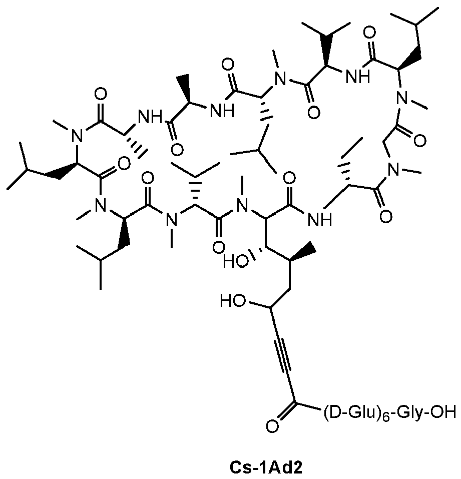

Ausführungsform (x): Verfahren zur Anreicherung eines Cyclosporinderivats in einem extrazellulären Raum eines multizellulären Objekts, umfassend die Schritte:
- Bereitstellen eines Cyclosporinderivats nach einer der Ausführungsformen (i) bis (ix);
- Inkontaktbringen des Cyclosporinderivats mit einem multizellulären Objekt.

Ausführungsform (xi): Verfahren nach Ausführungsform (x), dadurch gekennzeichnet, dass das multizelluläre Objekt ein separiertes Organ oder Körperteil, Blut oder eine Blutfraktion, eine Zellkultur oder eine Pflanze ist.

Ausführungsform (xii): Verwendung eines Cyclosporinderivats nach einer der Ausführungsformen (i) bis (ix) als Medikament.

Ausführungsform (xiii): Verwendung eines Cyclosporinderivats nach einer der Ausführungsformen (i) bis (ix) zur Herstellung eines Medikaments zur Behandlung einer chronischen entzündlichen Erkrankung.

Ausführungsform (xiv): Verwendung nach Ausführungsform (xiii), wobei die chronische entzündliche Erkrankung Asthma, rheumatoide Arthritis, Multiple Sklerose, Psoriasis oder ulzerative Kolitis ist.

Ausführungsform (xv): Pharmazeutische Zusammensetzung, umfassend ein Cyclosporinderivat nach einer der Ausführungsformen (i) bis (ix).

### Beispiele und Figuren

Die vorliegende Erfindung soll nun anhand der folgenden Beispiele und Figuren näher beschrieben werden. Die Beispiele und Figuren haben dabei rein veranschaulichenden Charakter und sollen den Rahmen der vorliegenden Erfindung keinesfalls einschränken.

Es zeigen
Fig. 1 den Einfluss von Cs-1O3 auf die Anzahl der CD4-positiven T-Zellen, welche durch die Ovalbuminsensibilisierung in die Bronchialschleimhäute einwanderten;
Fig. 2 den Einfluss von 250 µg Cs-1O3 auf die Anzahl der eosinophilen Granulozyten (Eosinophile), welche durch die Ovalbuminsensibilisierung in die Bronchialschleimhäute einwanderten;
Fig. 3 den Einfluss von 250 µg Cs-1O3 auf die Anzahl der Eosinophilen Granulozyten (Eosinophile), welche durch die Ovalbuminsensibilisierung in die Bronchialschleimhäute einwanderten und sich in der Lungenspülflüssigkeit (Lavage) nachweisen lassen;
Fig. 4 die Anzahl der Leukozyten im Blut vor und nach Verabreichung einer dreimaligen Dosis (1., 3. und 5. Tag) von 250 µg Cs-103 je Tier(Bestimmung am 6. Tag); und
Fig. 5 die Anzahl der Leukozyten in der Milz vor und nach Verabreichung einer dreimaligen Dosis (1., 3. und 5. Tag) von 250 µg Cs-1O3 je Tier(Bestimmung am 6. Tag).

### Beispiele

### Beispiel 1: Synthese von Cs-1H1

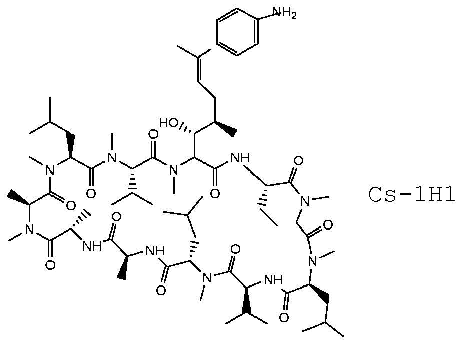

100 mg CsA (0,075 mmol) wurden in 11 ml einer Mischung von DMF/H₂O/Et₃N im Verhältnis von 8:1:1 unter Schutzgas (Argon) gelöst. Dann wurden jeweils 0,4 Äquivalente Pd(OAc)₂, PPh₃ und Bu₄NC1 und 20 Äquivalente p-Bromanilin zugesetzt. Nach Erhitzen der Mischung bei 103°C für 5 Stunden wurde die Mischung auf Raumtemperatur abgekühlt und 0,5 Äquivalente AcOEt wurden zugesetzt. Anschließend wurde dreimal mit Kochsalzlösung gewaschen. Danach wurde die AcOEt-Fraktion über MgSO₄ filtriert und im Vakuum eingedampft. Das Produkt wurde mittels präparativer HPLC von Verunreinigungen abgetrennt.

### Beispiel 2: Synthese von Cs-1WZ2

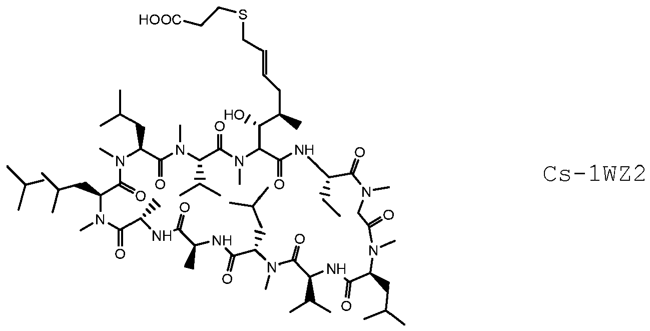

O-Azetyl-CsA wurde entsprechend einer Vorschrift von R. Traber et al. synthetisiert (Helv. Chim, Acta 1982, 65, 1655-1677). 50 mg O-Azetyl-CsA wurden in 20 ml CCl₄ gelöst. Anschließend wurden eine katalytische Menge von AIBN und 1 Äquivalent NBS zugesetzt. Danach wurde die Mischung für zwei Stunden unter Rückfluss gehalten. Anschließend wurde das Lösungsmittel verdampft und der Rückstand mit 100 ml ACOEt aufgenommen. Danach wurde die AcOEt-Lösung nacheinander mit 5%-iger KHSO₄-Lösung, gesättigter NaHCO₃-Lösung und Kochsalzlösung gewaschen und anschließend über MgSO₄ getrocknet. Nach Abfiltrieren und Eindampfen im Vakuum wurde der Rückstand in einer Lösung von 3 Äqivalenten Methyl-3-mercaptopropionat und 5 Äquivalenten Diisopropylethamin aufgenommen, worauf 5 ml DMF zugegeben wurden. Danach wurde für drei Stunden bei Raumtemperatur gerührt. Anschließend wurde DMF im Vakuum entfernt und dem Rückstand wurden 5 ml 0,1 M LiOH in 50%-igem Methanol zugesetzt. Nach drei Stunden wurde die Reaktion mittels HCl auf pH 2,5 gebracht und anschließend mittels präparativer HPLC das Produkt isoliert.

### Beispiel 3: Synthese von Cs-1AO2

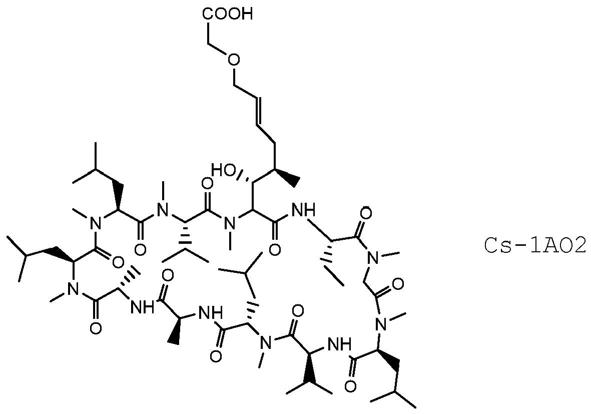

60 mg der Vorstufe Cs-1AO1 wurden entsprechend der Vorschrift von Eberle et al. (J. Org. Chem. 1992 , 57, 2689) hergestellt.

Danach wurde das Produkt mit einer Lösung aus 20 mg Butylbromazetat und 5 mg Benzyltriethylammoniumchlorid in 1 ml Dichlormethan aufgenommen, mit 2 ml 30%-iger NaOH-Lösung versetzt und zwei Stunden bei Raumtemperatur gerührt. Nach Verdünnen mit 10 ml Wasser wurde das Produkt zweimal mit Ether extrahiert. Nach Trocknen der organischen Phase mittels MgSO₄ und Filtrieren wurde im Vakuum eingedampft und der Rückstand mit 5 ml 0,1 M LiOH in 50%-igem Methanol versetzt und für drei Stunden bei Raumtemperatur gerührt. Nach Ansäuern mittels HCl auf pH 2,5 wurde das Produkt Cs-1A02 mittels präparativer HPLC isoliert.

### Beispiel 4: Herstellung von Cs-1O1

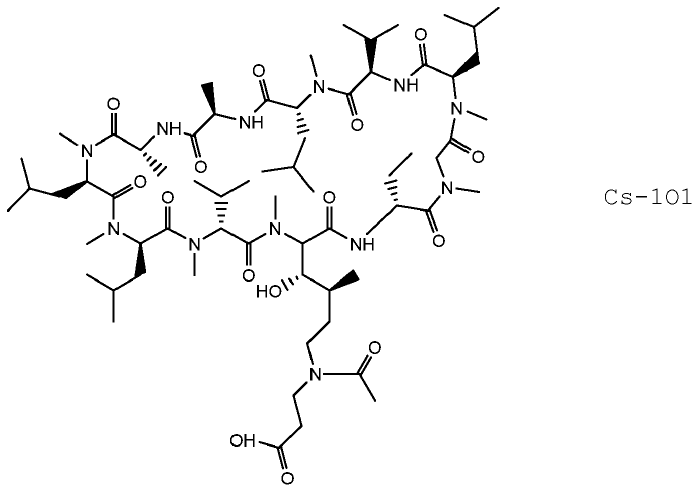

150 mg des Ausgangsproduktes O-Acetyl-CsA-Aldehyd wurde entsprechend J. Liu et al. (Anal. Biochem 2006, 356, 100-107) hergestellt und anschließend mit 250 mg H-β-Ala-OH in 30 ml Methanol versetzt. Nach Mischen für 10 Minuten wurden 8,5 mg NaBH₃CN, gelöst in 500 µl Methanol zugegeben und über Nacht gerührt. Nach Absenken des pH mit HCl auf etwa 1,5 wurde das Methanol im Vakuum abgedampft und der Rückstand in Kochsalzlösung aufgenommen und das erhaltene Gemisch dreimal mit ACOEt extrahiert. Anschließend wurde mit 5%-iger KHSO₄-Lösung, gesättigter NaHCO₃-Lösung und Kochsalzlösung gewaschen. Nach Trockenen über MgSO₄ und Filtrieren wurde im Hochvakuum getrocknet. Der Rückstand wurde anschließend in einer Mischung, bestehend aus 15,4 µl ACOH, 104 mg HATU und 145 µl DIPEA, und 1 ml DMF, aufgenommen. Nach Mischen für zwei Stunden bei Raumtemperatur wurde ACOEt zugegeben und nacheinander zweimal mit gesättigter NaHCO₃, 5%-iger KHSO₄ und Kochsalzlösung gewaschen. Nach Trocknen mit MgSO₄ und Filtrieren wurde das Lösungsmittel im Vakuum verdampft. Der Rückstand wurde in 3 ml THF, gemischt mit 3 ml 0,2 M LiOH, aufgenommen und 4 Stunden gerührt. Nach Ansäuern mit HCl auf einen pH von etwa 1,5 konnte das Produkt mittels präparativer HPLC isoliert werden.

### Beispiel 5: Herstellung von Cs-1O6

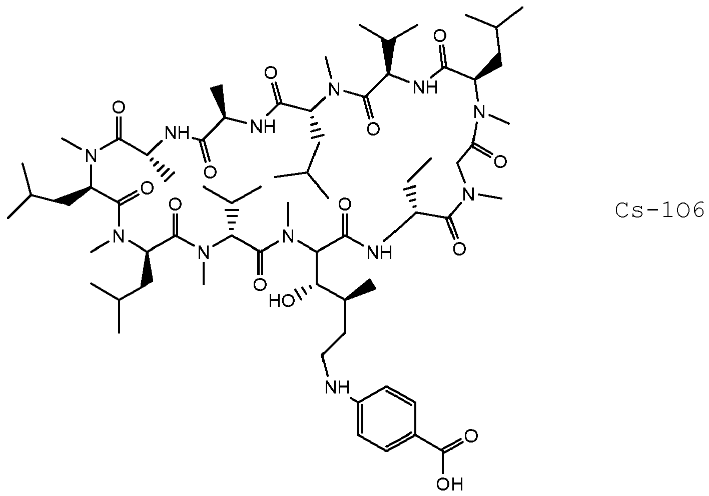

40 mg des Ausgangsproduktes O-Acetyl-CsA-Aldehyd wurden entsprechend der Vorschrift von J. Liu et al. (Anal. Biochem. 2006, 356, 100-107) hergestellt und anschließend in 10 ml Methanol gelöst. Zur Lösung wurden anschließend 22 mg 4-Aminobenzoesäure und 3 mg NaBH₃CN zugegeben. Nach Rühren über Nacht bei Raumtemperatur wurde die Lösung mittels HCl auf einen pH von 1,5 eingestellt. Anschließend wurde das Methanol verdampft und der Rückstand mit Kochsalzlösung aufgenommen. Danach wurde dreimal mit ACOEt extrahiert und die vereinigten Extrakte nacheinander mit 5%-iger KHSO₄, gesättigter NaHCO₃ und Kochsalzlösung gewaschen. Nach Trocknen über MgSO₄ und Filtrieren wurde im Vakuum eingedampft. Der Rückstand wurde in einer Mischung aus 3 ml THF und 3 ml 0,2 M LiOH aufgenommen und 4 Stunden gerührt. Nach Ansäuern mittels HCl auf einen pH von etwa 1,5 wurde das Produkt mittels präparativer HPLC isoliert.

### Beispiel 6: Herstellung von Cs-1O11

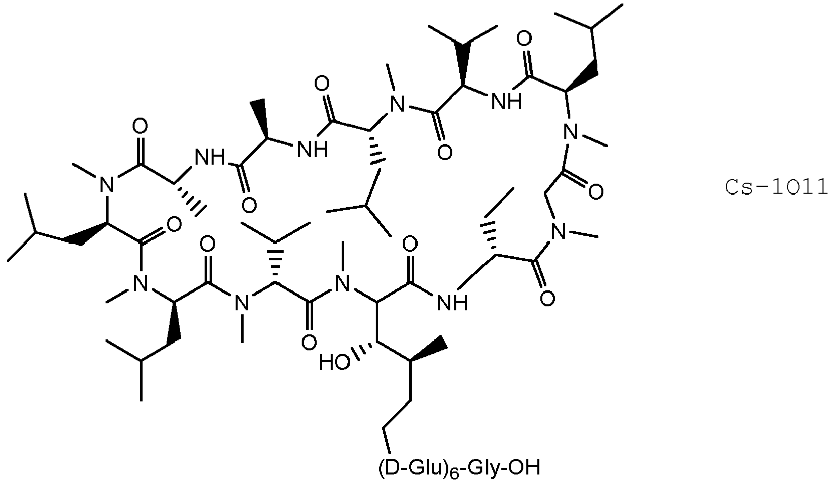

40 mg des Ausgangsproduktes O-Acetyl-CsA-Aldehyd wurden entsprechend der Vorschrift von J. Liu et al. (Anal. Biochem. 2006, 356, 100-107) hergestellt und anschließend in 10 ml Methanol gelöst. Zur Lösung wurden danach 3 Äquivalente H-(D-Glu)₆-Gly-OH (synthetisiert mittels Festphasen-Peptid-Synthese, ausgehend von D-Glu(Ot-Bu)-OH)und 3 mg NaBH₃CN gegeben und über Nacht bei Raumtemperatur gerührt. Danach wurde die Lösung mittels HCl auf einen pH von 1,5 eingestellt. Anschließend wurde das Methanol verdampft und der Rückstand in einer Mischung von 3 ml THF und 3 ml 0,2 M LiOH aufgenommen. Danach wurde 4 Stunden bei Raumtemperatur gerührt. Nach Ansäuern mittels HCl auf einen pH von etwa 1,5 wurde das Produkt mittels präparativer HPLC isoliert.

### Beispiel 7: Herstellung von Cs-1S1

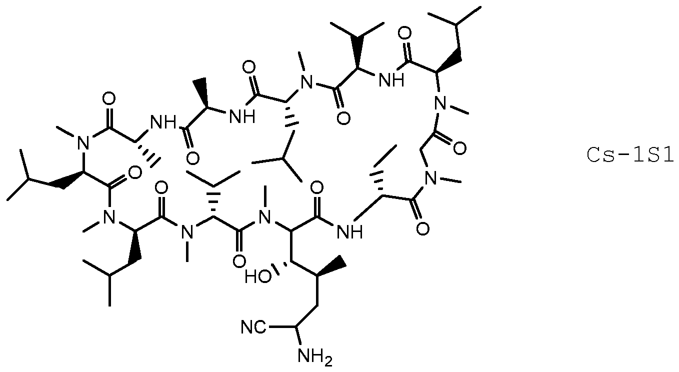

50 mg des Ausgangsproduktes O-Acetyl-CsA-Aldehyd wurden entsprechend der Vorschrift von J. Liu et al. (Anal. Biochem. 2006, 356, 100-107) hergestellt und anschließend in 2 ml Methanol gelöst. Zur Lösung wurde anschließend bei 5 °C langsam eine Lösung von 3 mg NH₄Cl und 3 mg KCN, gelöst in 25%-igem NH₃, gegeben. Nach 4 Stunden Rühren bei Raumtemperatur wurde im Vakuum eingedampft und das Produkt mittels präparativer HPLC isoliert.

### Beispiel 8: Herstellung von Cs-1Ad1

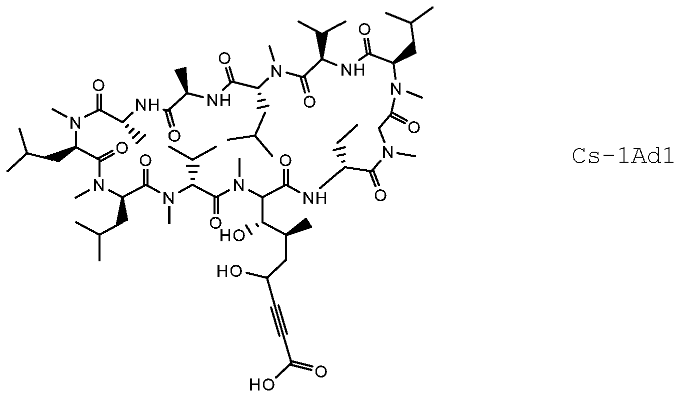

60 mg des Ausgangsproduktes O-Acetyl-CsA-Aldehyd wurden entsprechend der Vorschrift von J. Liu et al. (Anal. Biochem. 2006, 356, 100-107) hergestellt und anschließend in THF gelöst und auf -78 °C abgekühlt. Separat wurde eine Mischung aus Diisopropylamin in THF und N-Butyllithium 30 min bei - 78 °C gerührt. Beide Mischungen wurden bei -78 °C vereinigt und bis zum Erreichen von Raumtemperatur gerührt. Anschließend wurde der THF-Überstand im Vakuum entfernt und der Rückstand in ACOEt aufgenommen. Der ACOEt-Extrakt wurde zweimal nacheinander mit 5%-iger KHSO₄-Lösung, gesättigter NaHCO₃-Lösung und Kochsalzlösung gewaschen. Nach Trocknen über MgSO₄ und Filtrieren wurde im Vakuum eingedampft. Der Rückstand wurde in einer Mischung aus 3 ml THF und 3 ml 0,2 M LiOH aufgenommen und 4 Stunden gerührt. Nach Ansäuern mittels HCl auf einen pH von etwa 1,5 wurde das Produkt mittels präparativer HPLC isoliert.

### Beispiel 9: Herstellung von CsM4

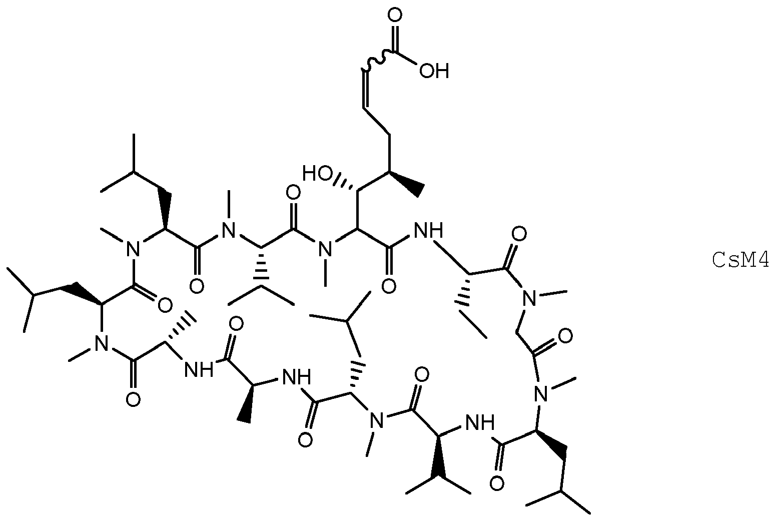

Cyclosporin A (CsA) wurde mit 0,1 Äquivalenten Hoveyda-Grubbs-Katalysator (II. Generation) und 20 Äquivalenten Diethylmaleat in Toluol 45 Stunden unter Rückfluß gehalten. Nach Entfernen des Toluols im Vakuum wurde der Rückstand in DCM/MeOH (10:0.5) gelöst und durch Silicagel filtriert. Nach Entfernen des organischen Lösungsmittels im Vakuum wurde der Rückstand in 5 ml THF gelöst und mit einer 0,2 M LiOH-Lösung versetzt. Nach Rühren über Nacht und Neutralisieren der Lösung mit HCl konnte das Produkt mittels präparativer HPLC abgetrennt werden.

### Beispiel 10: Herstellung von Cs-1OM3

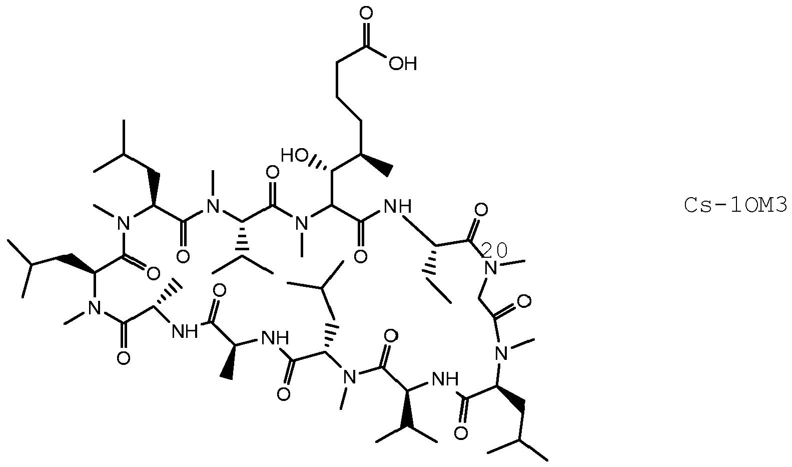

Cyclosporin A (CsA) wurde mit 0,1 Äquivalenten Hoveyda-Grubbs-Katalysator (II. Generation) und 20 Äquivalenten Diethylmaleat in Toluol 45 Stunden unter Rückfluß gehalten. Nach Entfernen des Toluols im Vakuum wurde der Rückstand in DCM/MeOH (10:0.5) gelöst und durch Silicagel filtriert. Nach Entfernen des organischen Lösungsmittels im Vakuum wurde der Rückstand in 5 ml Methanol gelöst und Palladium-Aktivkohle-Katalysator unter H₂ zugegeben. Nach Hydrierungsende wurde filtriert und 2 ml THF sowie 2 ml 0,2 M LiOH-Lösung wurden zugegeben. Nach Rühren über Nacht wurde mit HCl neutralisiert und das Produkt mittels präparativer HPLC abgetrennt.

### Beispiel 11: Herstellung von Cs-1E1

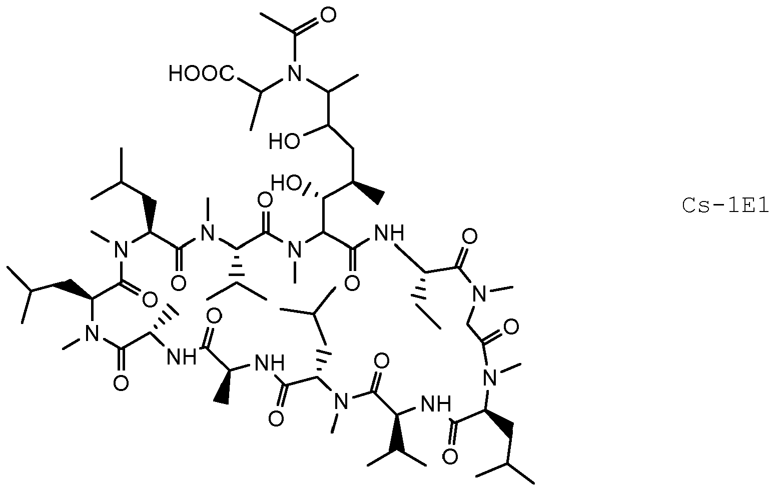

O-Azetyl-CsA wurde entsprechend einer Vorschrift von R. Traber et al. (Helv. Chim. Acta 1982, 65, 1655-1677) synthetisiert und anschließend in Dichlormethan (DCM) gelöst. Nach Zugabe von 1,1 Äquivalenten m-Chloroperbenzoesäure wurde zwei Stunden bei Raumtemperatur gerührt. Anschließend wurde das DCM im Vakuum entfernt und der Rückstand mit 10 Äquivalenten H-Ala-Ot-Bu und DMF aufgenommen und bei 50 °C 5 Stunden gerührt. Im Vakuum wurde anschließend das DMF entfernt und der Rückstand mit AcOEt aufgenommen. Der AcOEt-Extrakt wurde zweimal nacheinander mit 5%-iger KHSO₄-Lösung, gesättigter NaHCO₃-Lösung und Kochsalzlösung gewaschen. Nach Trocknen über MgSO₄ und Filtrieren wurde im Vakuum eingedampft und im Hochvakuum getrocknet. Der Rückstand wurde danach mit voraktivierter Essigsäure (drei Äquivalente Essigsäure, drei Äquivalente HATU, 6 Äquivalente DIPEA in DMF; insgesamt 5 Minuten aktiviert) versetzt und für zwei Stunden bei Raumtemperatur gerührt. Anschließend wurde DMF im Vakuum entfernt und der Rückstand mit ACOEt aufgenommen. Anschließend wurde der ACOEt-Extrakt nacheinander mit 5%-iger KHSO₄-Lösung, gesättigter NaHCO₃-Lösung und Kochsalzlösung gewaschen. Nach Trocknen über MgSO₄ und Filtrieren wurde im Vakuum eingedampft und im Hochvakuum getrocknet. Dann wurde der Rückstand mit 0,1 M LiOH bis zum Ende der Hydrolyse gerührt. Das Produkt wurde anschließend mittels präparativer HPLC isoliert.

### Beispiel 12: Herstellung von Cs-1H2

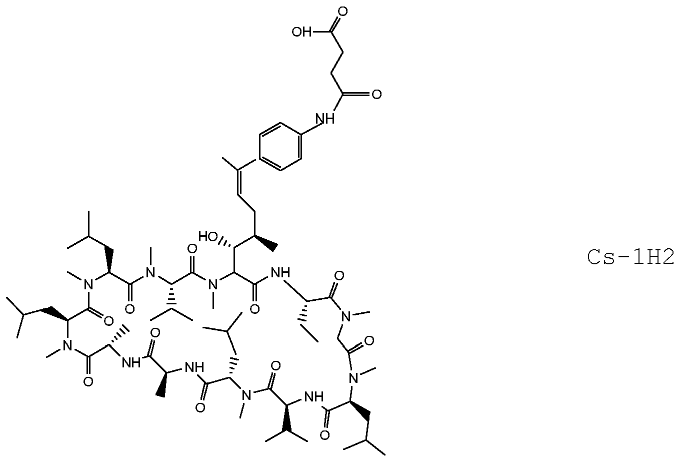

Zu einer Lösung von 8 mg Cs-1H1 (Beispiel 1) in 1 ml DMF wurden 80 mg Bernsteinsäureanhydrid und 223 µl Triethylamin gegeben. Diese Mischung wurde bei Raumtemperatur zwei Tage gerührt. Das Produkt Cs-1H2 konnte direkt aus dem Ansatz mittels präparativer HPLC isoliert werden.

### Beispiel 13: Herstellung von Cs-1H2-2

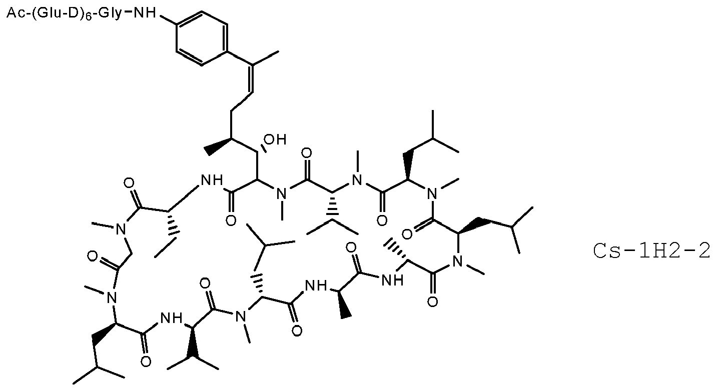

100 mg Ac-(D-Glu(OMe))₆-Gly-OH (synthetisiert mittels Festphasen-Peptidsynthese) wurden in 5 ml DMF gelöst und mit 1,1 Äquivalenten HATU und 3 Äquivalenten DIPEA 5 Minuten aktiviert. Anschließend wurde Cs-1H1 (Beispiel 1) zugegeben. Nach Rühren des Ansatzes für zwei Stunden bei Raumtemperatur wurde das Lösungsmittel im Vakuum entfernt. Zum Rest wurden 0,1 M LiOH, gelöst in 50%-igem Methnaol zugegeben und gemischt bis der Methylester vollständig hydrolysiert war. Das Endprodukt (Cs-1H2-2) konnte mittels präparativer HPLC erhalten werden.

### Beispiel 14

Mit dem Verfahren von Beispiel 13, wobei entsprechend andere Edukte eingesetzt wurden, wurden auch die nachstehend gezeigten Cyclosporinderivate Cs-1E3, Cs-1S2 und Cs-1O9 erhalten:

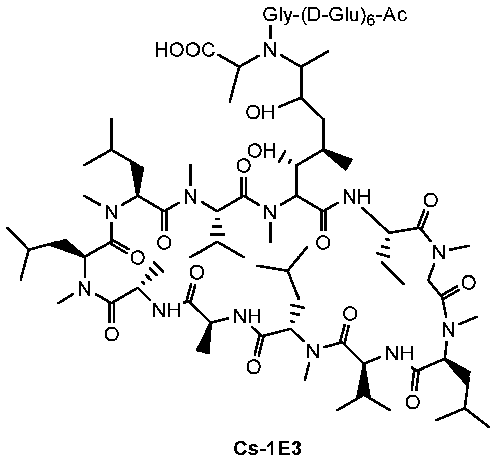

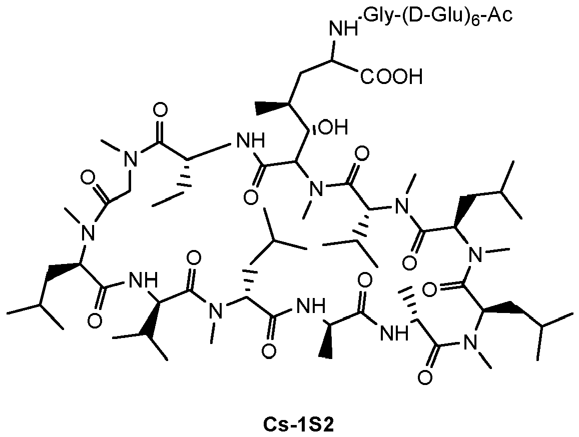

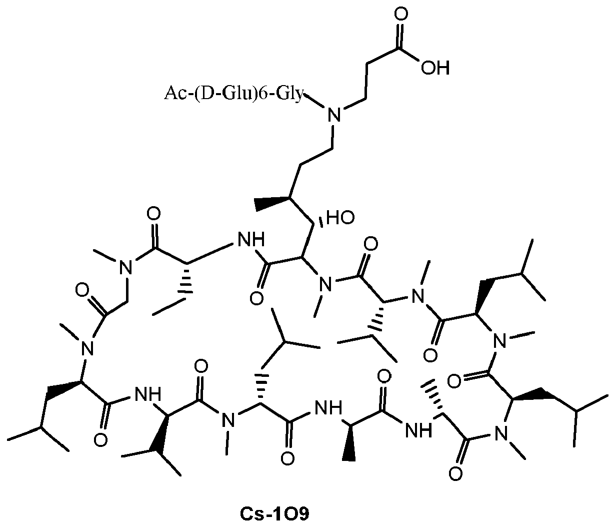

### Beispiel 15

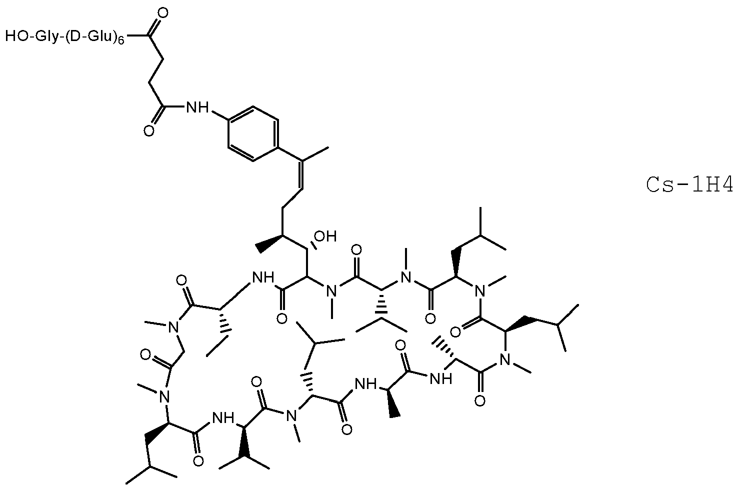

50 mg Cs-1H2 (Beispiel 12), ein Äquivalent HATU und drei Äquivalente DIPEA in 3 ml DMF wurden 20 min bei Raumtemperatur gerührt. Anschließend wurde 1 Äquivalent H-(D-Glu)₆-Gly-OH (synthetisiert mittels Festphasen-Peptidsynthese) gelöst in 2 ml DMF zugegeben und die Lösung über Nacht gerührt. Nach dem Filtrieren konnte das Produkt mittels präparativer HPLC isoliert werden.

Mit dem Verfahren von Beispiel 13, wobei entsprechend andere Edukte eingesetzt wurden, wurden auch die nachstehend gezeigten Cyclosporinderivate Cs-1WZ3, Cs-1AO3, Cs-1O3, Cs-1O7, Cs-M5, Cs-1OM4, Cs-1E2, Cs-3E2, Cs-3E6 und Cs-1Ad2 erhalten werden:

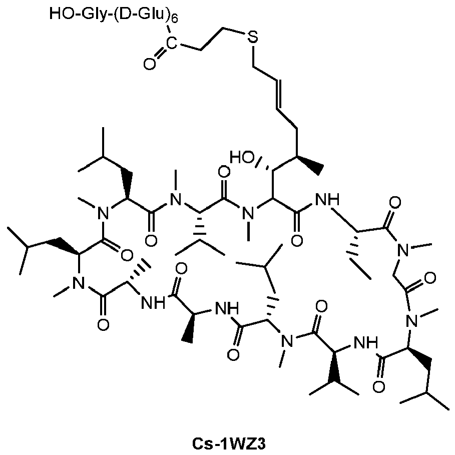

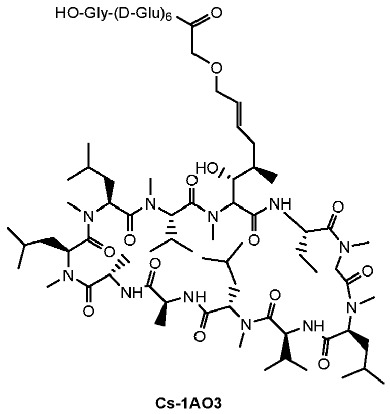

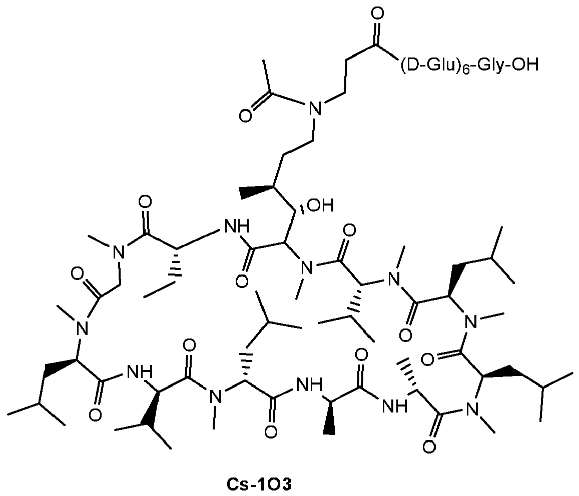

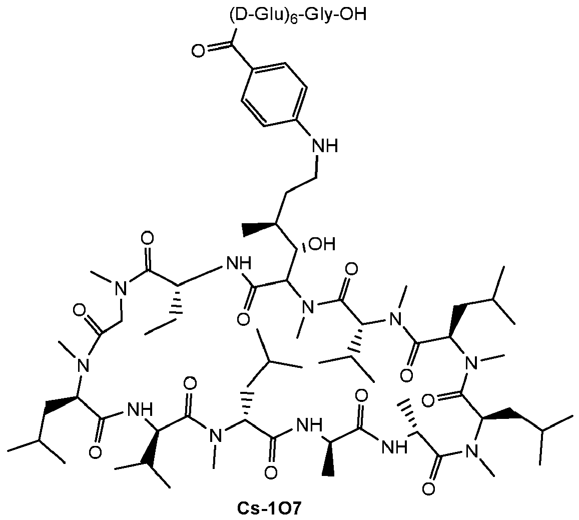

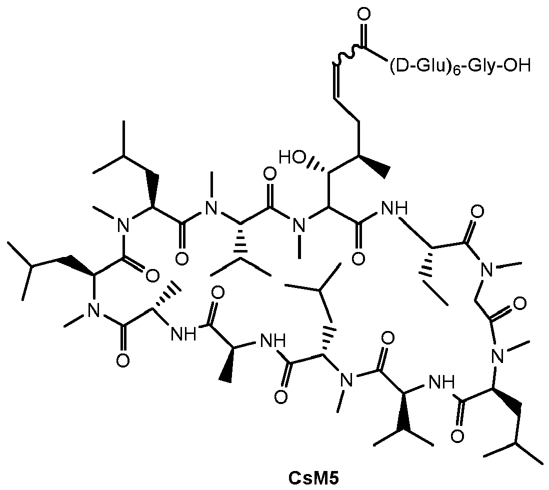

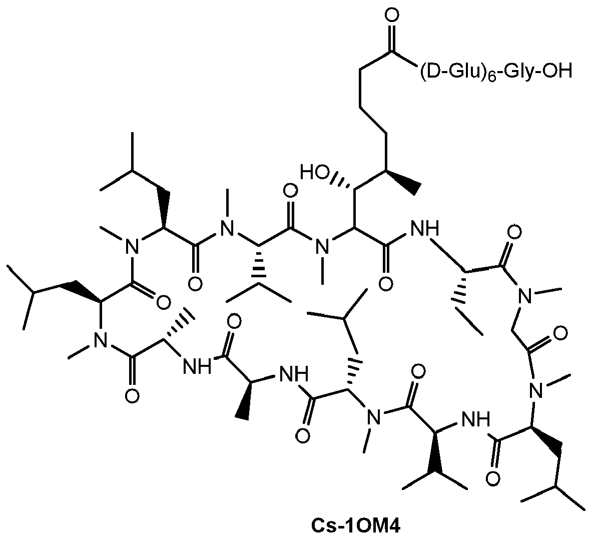

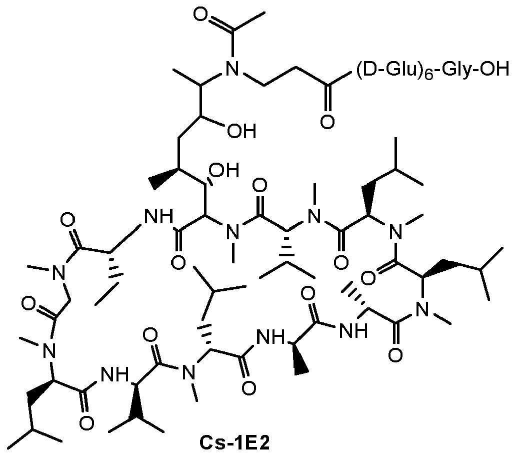

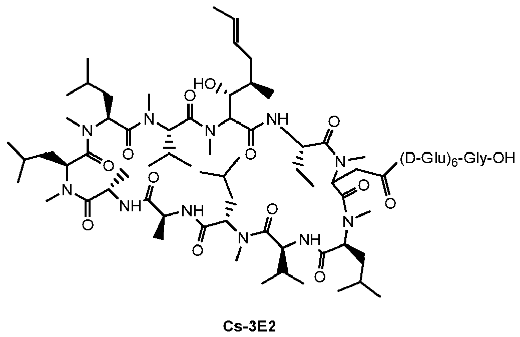

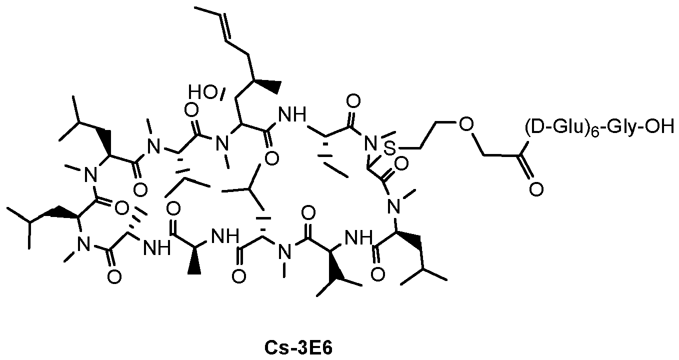

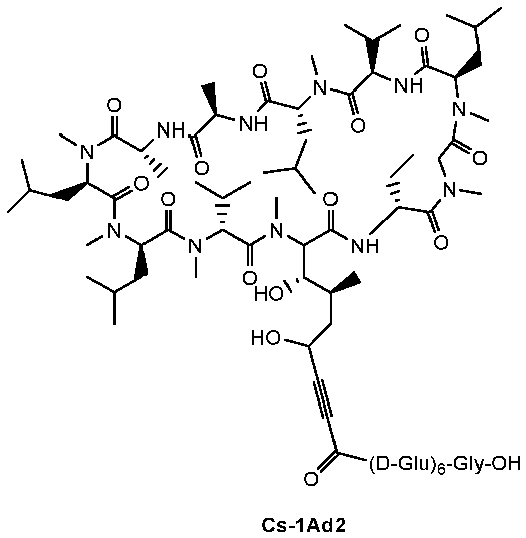

### Beispiel 16

### Herstellung von TAMRA-markiertem Cs-1O1

### 16a

TAMRA markierter trifunktionaler Linker MM-50

Zu einer Lösung von 5 (6)-Carboxytamra (130 mg) in 5 ml DMF wurden 114 mg HATU, 154 µl DIPEA und 82 mg HOAt zugegeben. Anschließend wurde die Lösung für 20 Minuten bei Raumtemperatur gemischt und zu einer Lösung von 200 mg des trifunktionalen Linkers MM-50 (Malesevic M, Lücke C, Jahreis G (2005) Simple and efficient synthesis of new trifunctional templates). Peptides 2004, Proceedings of the Third International and Twenty-Eighth European Peptide Symposium, Kenes International, Israel, 391-392) in 5 ml DMF zugegeben. Nach Mischen der Lösung für zwei Stunden bei Raumtemperatur und Filterung wurde DMF im Vakuum entfernt. Nach Abtrennung des Produktes mittels RP HPLC konnte eine Masse von 1105.2 [M+H]⁺ (theoretisch m/z cal. 1104.5) mittels MALDI-Massenspektrometrie bestimmt werden.

### 16b

Kupplung des TAMRA markierten trifunktionellen Linkers an Cs-101

50 mg Cs-101 (Beispiel 4), ein Äquivalent HATU und drei Äquivalente DIPEA in 3 ml DMF wurden 20 min bei Raumtemperatur gerührt. Anschließend wurde 1 Äquivalent des trifunktionellen Linkers gelöst in 2 ml DMF zugegeben und die Lösung über Nacht gerührt. Nach dem Filtrieren konnte das Produkt mittels präparativer HPLC isoliert werden.

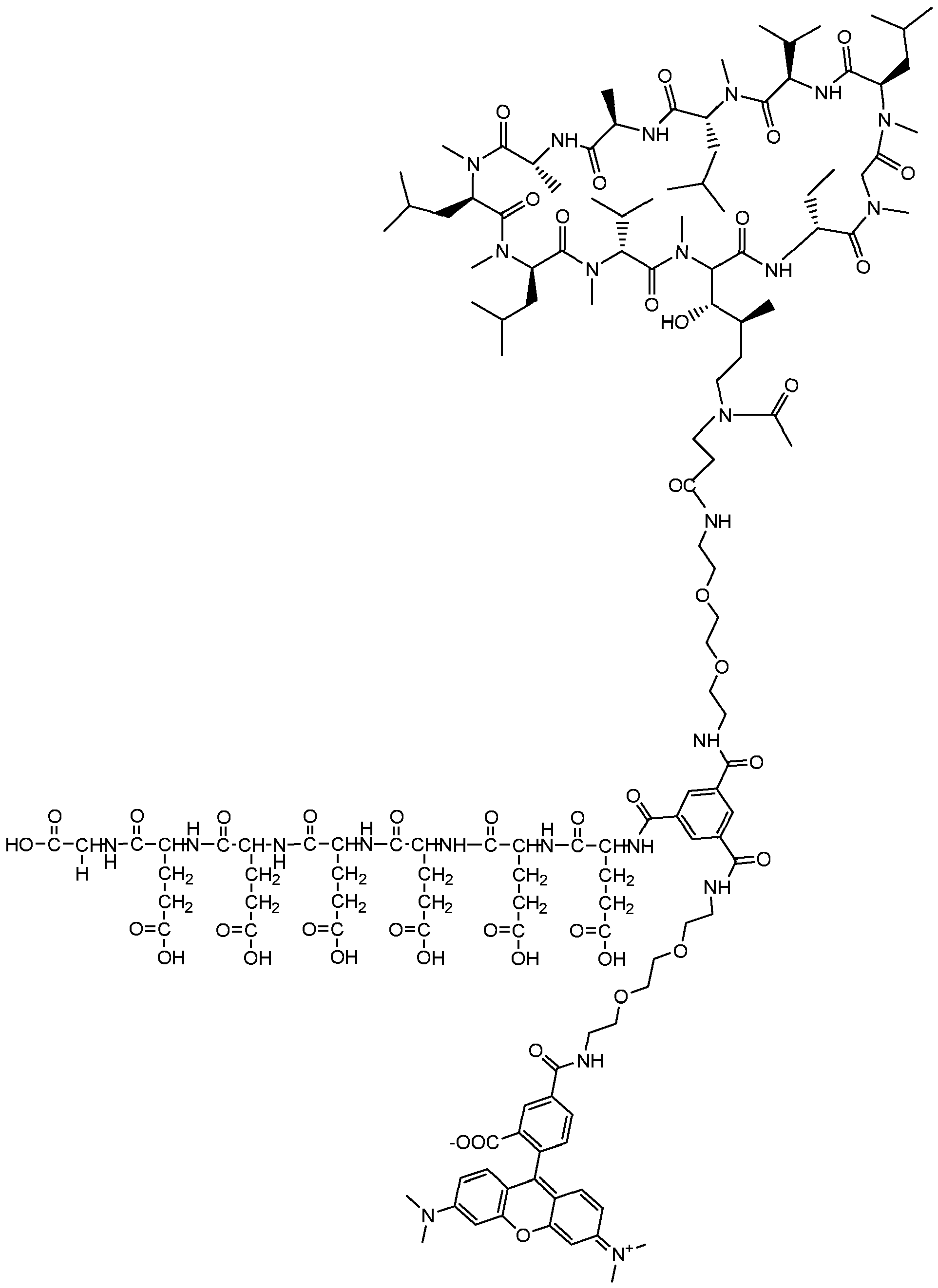

### Beispiel 17

Aufnahme von chemisch modifizierten CsA-Verbindungen durch Hela Zellen

Der Vorteil vorliegender Entdeckung wird bei der Untersuchung des Transportverhaltens des Cyclosporin-Derivates TAMRA-Cs-1S2 deutlich. Die Aufnahme von chemisch modifizierten, fluoreszierenden CsA-Derivaten in lebende eukaryontische Zellen wird an einer Zelllinie mittels Konfokaler-Laserscan-Mikroskopie gezeigt. Für denVersuch wurden 10⁵ Hela-Zellen in Petrischalen der Firma Ibidi® (µ-Dish, 35mm, high) eingesät und 1-2 Tage in DME-Medium (high Glucose) bei 37°C und 5% CO₂ inkubiert.

Die Untersuchung erfolgte an einem inversen Mikroskop (Nikon ECLIPSE C1TE2000-E), das mit einer Fokussierhilfe, dem T-PFS, ausgestattet ist, um einen sogenannten Focus-Drift zu verhindern. Für die Aufnahmen wurde ein Objektiv mit Phasenkontrast (40,0x Plan Fluor Oilimmersion NA 1,30) verwendet sowie die Mikroskop eigene Software EZ-C1 3.7. Die Anregung des Fluorophor 5-(6) Carboxytetramethylrhodamin erfolgte durch einen 561nm Laser von Melles & Griot.

Die Zellen wurden zunächst zweimal mit 2ml PBS pH 7,4 (Dulbecco) gewaschen und anschließend in 2ml MIK-Medium aufgenommen (Phenolrot-freies DME-Medium, FCS-frei, mit 20mM HEPES pH 7,2 und 0,01% Carbencilin) und für 20min bei 37°C und 5% CO₂ inkubiert. Während der Aufnahmen waren die Zellen in einem Inkubator für Mikroskope (Stage Top Incubator INU series von Tokai Hit®) bei 37°C und 5% CO₂. Die Untersuchung wurde durch die Zugabe von in DMSO gelöstem und in MIK-Medium verdünntem 250nM Cs1-Derivat gestartet. Im Fluoreszenzlicht sind die Hela Zellen als fluoreszierende Zellen sichtbar. Das mit einem sauren Peptid versehene Cyclosporinderivat reichert sich nicht innerhalb der Hela-Zellen an.

### Beispiel 18

### a) Präparation und Herstellung mononuklearer Zellen von Mäusen

Eine operativ entfernte Milz von Mäusen (BALB/c-Linie) wurde zwischen Objektgläsern gequetscht, um geeignete Zellsuspensionen herzustellen. Anschließend wurde die so erhaltene Suspension durch ein Nylonsieb filtriert, um grobe Bestandteile abzutrennen. Die so erhaltenen Zellen wurden zusammen mit einem Lymphozytentrennmedium (Mediatech) zentrifugiert, um mononukleare Zellen zu erhalten.

Anschließend wurden Zellkulturen dieser Zellen in Mikrotiterplatten (8 x 12 Kavitäten) bei einer Zelldichte von 6 x 10⁵ Zellen je Kavität in EHAA Medium/5% FCS (Clicks Medium) in der Gegenwart von 10 µg/ml Concanavalin A (ConA), mit 2 µM of Cs-103, mit unmodifiziertem Cyclosporin A (Sigma) oder mit 1% Ethanol (Verdünnungsmittel) inkubiert. Nach einer Kulturzeit von 48 Stunden wurde 1 µCi 3H-Thymidin je Kavität zugegeben und für weitere 6 Stunden inkubiert. Anschließend wurden die Zellen jeder Kavität geerntet (TomTec 96-well Harvester) und die Radioaktivität der Zellen gemessen (Tri-Lux beta-plate Counter).

### b) Asthma-Untersuchungen

Durch Gabe von Ovalbumin zusammen mit Aluminiumhydroxid wird eine Immunantwort gegen Ovalbumin provoziert. Die Immunantwort kann anhand der in die Bronchialschleimhaut eingewanderten T-Helferzellen-Population (CD4+) und der eingewanderten eosinophilen Granulozyten verfolgt werden.

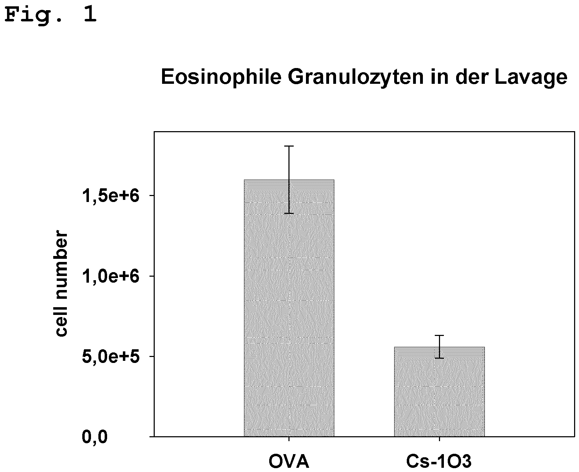

Weibliche Mäuse (BALB/c-Linie) wurden durch intraperitoneale (i.p.) Gabe von 50 µg Ovalbumin in Phosphatpuffer (PBS) gelöst (OVA) plus 100 µL Aluminiumhydroxid (alum) bei einem Gesamtvolumen von 200 µL je Maus am Tag 0 sensibilisiert. Den OVA/alum-sensibilisierten Mäusen wurde dann unter milder Anästhesie (Isofluran) an den Tagen 7 bis 10 intranasal jeweils 100 µg OVA in PBS (50 µL Gesamtvolumen) verabreicht. Aus diesen Tieren wurden Gruppen gebildet, welche zusätzlich an den Tagen 7, 9 und 11 entweder 250 µg Cs103 in PBS (i.p.), nur PBS (Verdünnungsmittel) oder keinen weiteren Zusatz (-) erhielten. Am Tag 12 wurden alle Tiere durch CO₂-Exposition getötet und Zellen des Bronchialtraktes durch Bronchiallavage (BAL) mittels einer in die Trachea eingeführten Kanüle bei dreimaligem Wasche mit je 1 ml kaltem PBS erhalten. Die erhaltenen Zellen der BAL wurden dann doppelt angefärbt (a) mit Cy-Chrome-konjugierten anti-Maus-CD4-Antikörpern und (b) mit FITC-konjugierten anti-Maus-CD62L-Antikörpern. Anschliessend wurden die Zellen mittels FACS analysiert. Effector/Memory-CD4⁺-T-cells wurden als CD4⁺/CD62L⁻-Lymphocyten und eosinophile Zellen anhand ihrer Streulichteigenschaften (FSC/SSC) unterschieden. Die Ergebnisse sind in den Figuren 1 bis 3 zusammengefasst.

Die Fig. 1 zeigt den Einfluss von Cs-1O3 auf die Anzahl der CD4-positiven T-Zellen, welche durch die Ovalbuminsensibilisierung in die Bronchialschleimhäute einwanderten. Die mit OVA sensibiliserten Tiere dienten als Kontrolle. Aus der Fig. 1 ist ersichtlich, dass die Gabe von Cs-1O3 die Anzahl der CD4 positiven T-Zellen sehr signifikant reduzierte.

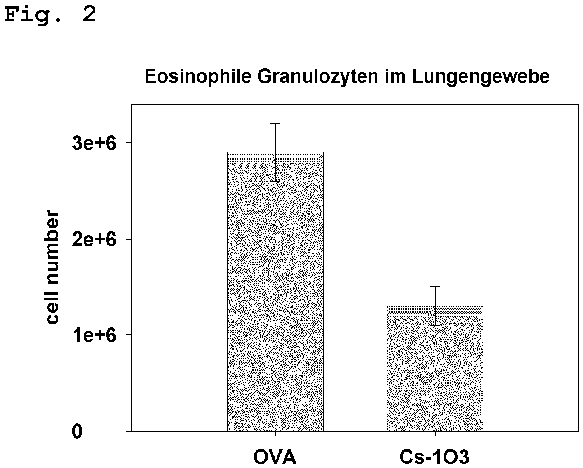

Die Fig. 2 zeigt den Einfluss von 250 µg Cs-1O3 auf die Anzahl der eosinophilen Granulozyten (Eosinophile), welche durch die Ovalbuminsensibilisierung in die Bronchialschleimhäute einwanderten. Die mit OVA sensibiliserten Tiere dienten als Kontrolle. Aus der Fig. 1 ist ersichtlich, dass die Gabe von Cs-1O3 die Anzahl der eosinophilen Granulozyten sehr signifikant reduzierte.

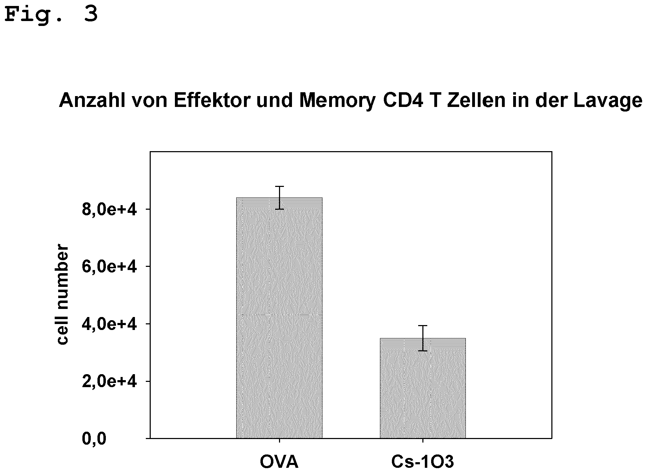

Zur Kontrolle wurde die Anzahl der eosinophilen Granulozyten in der Lungenspülflüssigkeit (Lavage) bestimmt (vgl. Fig. 3). Wie es aus der Fig. 3 ersichtlich ist, reduzierte auch hier die Gabe von 250 µg Cs-1O3 die Anzahl der eosinophilen Granulozyten sehr signifikant.

### b) Toxizitätstest

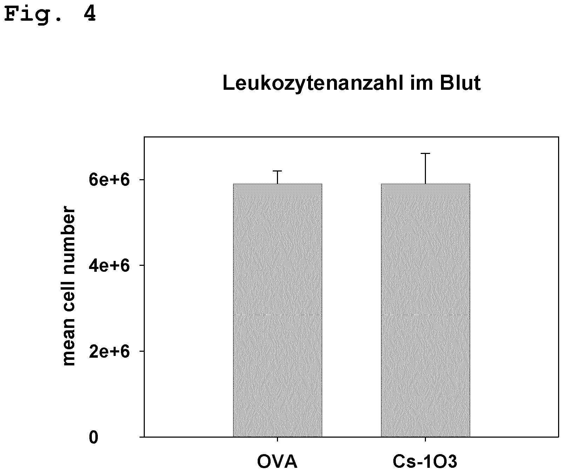

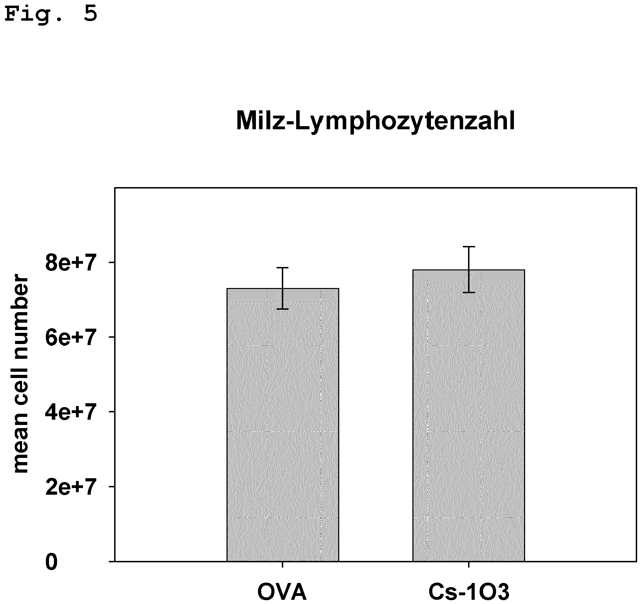

Da bei entzündlichen Erkrankungen die Leukozytenzahl in Blut und Milz ansteigt, wurde die Zahl dieser Zellen als Toxizitätsmarker genutzt. Wie es die Figuren 4 und 5 zeigen, ist die ausgewählte Verbindung Cs-1O3 (nach dreimaliger Gabe von 250 µg Cs-103 am 1., 3. und 5. Tag und der Zellzahlbestimmung am 6. Tag) in diesem Assay unbedenklich.

## Patentansprüche

1. Cyclosporinderivat der allgemeinen Formel (I) 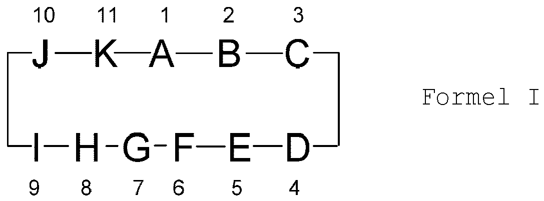 oder ein pharmazeutisch verträgliches Salz davon, wobei in Formel (I)
A ein Aminosäurerest der Formel (II) ist, 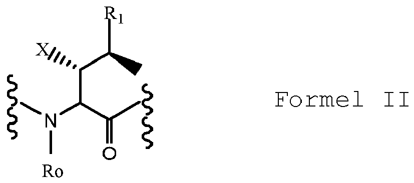 bei dem R₁ einem Rest der Formel III 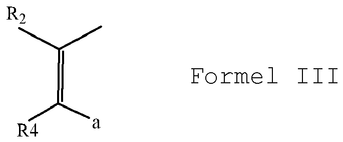 entspricht, der über das Kohlenstoffatom a kovalent verknüpft ist, oder
bei dem R₁ einem Rest der Formel V 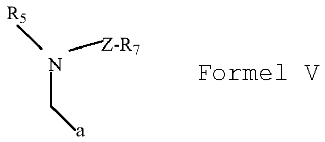 entspricht, der über das Kohlenstoffatom a kovalent verknüpft ist, oder
bei dem R₁ einem Rest der Formel VIII 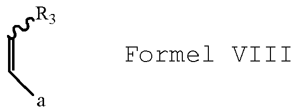 entspricht, der über das Kohlenstoffatom a kovalent verknüpft ist,
wobei die Gruppe C=O in der Formel II über eine Amidbindung kovalent an einer alpha-Aminogruppe von B gebunden ist und die Gruppe N-Rₒ in der Formel II über eine Amidbindung kovalent an einer Carboxylgruppe von K gebunden ist,
B ein Aminosäurerest ist, der aus der Gruppe bestehend aus:
alpha-Aminobuttersäure,
Alanin,
Threonin,
Valin,
Norvalin und
in der Seitenkette mit einer Hydroxylgruppe
modifizierter alpha-Aminobuttersäure, Alanin, Threonin, Valin oder Norvalin
ausgewählt ist,
C ein Sarkosinrest ist,
D ein Aminosäurerest ist, der aus der Gruppe bestehend aus:
Leucin,
N-Methylleucin,
Valin,
gamma-Hydroxy-N-methylleucin und
gamma-Hydroxyleucin
ausgewählt ist,
E ein Aminosäurerest ist, der aus der Gruppe bestehend aus:
Valin,
Norvalin und
einem modifizierten Valin oder Norvalin, bei dem ein Kohlenstoffatom der Seitenkette mit einer
Hydroxylgruppe substituiert ist,
ausgewählt ist,
F ein Aminosäurerest ist, der aus der Gruppe bestehend aus:
Leucin,
N-Methylleucin,
gamma-Hydroxy-N-methylleucin und
gamma-Hydroxyleucin
ausgewählt ist,
G ein alpha-Aminobuttersäurerest oder ein Alaninrest ist, H ein D-Alaninrest ist,
I und J Reste sind, die unabhängig voneinander aus der Gruppe bestehend aus:
Leucin,
N-Methylleucin,
gamma-Hydroxy-N-methylleucin und
gamma-Hydroxyleucin
ausgewählt sind, und
K ein N-Methylvalinrest oder ein Valinrest ist,
wobei in den Formeln III, V und XIII
Rₒ für H oder CH₃ steht,
X für H oder
Hydroxyl oder
eine Hydroxylgruppe steht, die mit einer Alkanoyl-, Aryloyl-, Alkylaminocarbonyl-, Arylaminocarbonyl-, Arylalkylaminocarbonyl-, Alkoxycarbonyl-, Aryloxycarbonyl-, Arylalkyloxycarbonylgruppe oder einer Silylschutzgruppe derivatisiert ist,
R₂ für einen substituierten oder unsubstituierten
monozyklischen, bizyklischen oder trizyklischen Aryl- oder Heteroarylring, der mit R₃ substituiert ist, steht,
R₃ für -Z-CO-Y oder -CO-Y steht,
R₄ für H oder einen substituierten oder unsubstituierten
monozyklischen, bizyklischen oder trizyklischen Aryl- oder Heteroarylring, der mit R₃ substituiert ist, steht,
Z aus der Gruppe bestehend aus:
einer unverzweigten, verzweigten oder zyklischen Alkylgruppe mit 1 bis 20 Kohlenstoffatomen,
einer unverzweigten, verzweigten oder zyklischen Alkenylgruppe mit 1 bis 20 Kohlenstoffatomen und
einer unverzweigten, verzweigten oder zyklischen Alkinylgruppe mit 1 bis 20 Kohlenstoffatomen ausgewählt ist,
Y aus der Gruppe bestehend aus:
den geschützten oder ungeschützten Aminosäureresten Gly, D-Glu, D-Asp, D- und L-Cys(SO₃H), und
ungeschützten Peptidresten mit einer Kettenlänge von 2 bis 200 Aminosäureresten, enthaltend eine Aminosäureart oder eine Kombination von verschiedenen Aminosäurearten ausgewählt aus Gly, D-Glu, D-Asp, D- und L-Cys(SO₃H) ausgewählt ist,
R₅ aus der Gruppe bestehend aus:
einer Alkyl-, Alkenyl-, Alkylaminocarbonyl-, Alkylcarbonyl-, Alkoxycarbonyl-, den Aminosäureresten Gly, D-Glu, D-Asp, D- und L-Cys(SO₃H), und
Peptidresten mit einer Kettenlänge von 2 bis 200 Aminosäureresten, enthaltend eine Aminosäureart oder
eine Kombination von verschiedenen Aminosäurearten,
ausgewählt aus Gly, D-Glu, D-Asp, D- und L-Cys(SO₃H) ausgewählt ist,
R₇ aus der Gruppe bestehend aus:
einer Carboxyl-, Amino-, Thiol-, Hydroxylgruppe,
den geschützten oder ungeschützten Aminosäureresten Gly, D-Glu, D-Asp, D- und L-Cys(SO₃H), und
Peptidresten mit einer Kettenlänge von 2 bis 200 Aminosäureresten, enthaltend eine Aminosäureart oder
eine Kombination von verschiedenen Aminosäurearten ausgewählt aus Gly, D-Glu, D-Asp, D- und L-Cys(SO₃H),
ausgewählt ist.

2. Cyclosporinderivat nach Anspruch 1, ausgewählt aus den folgenden Verbindungen:

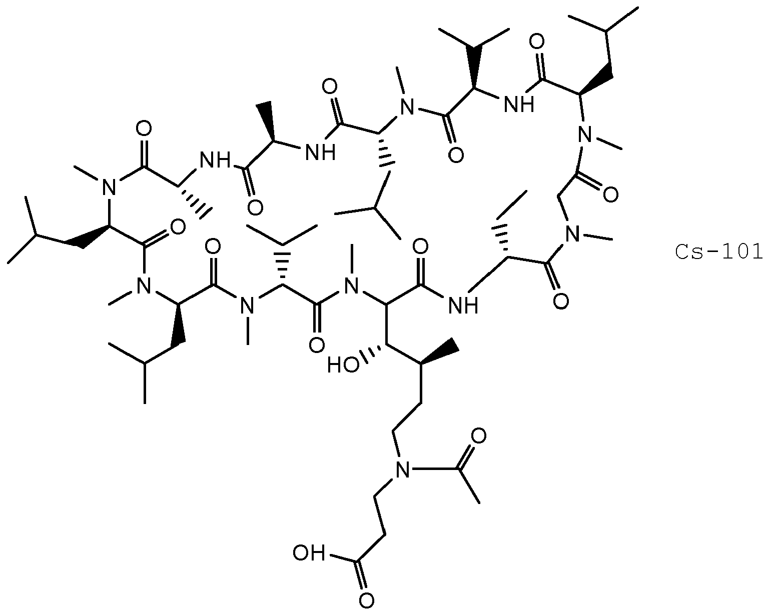

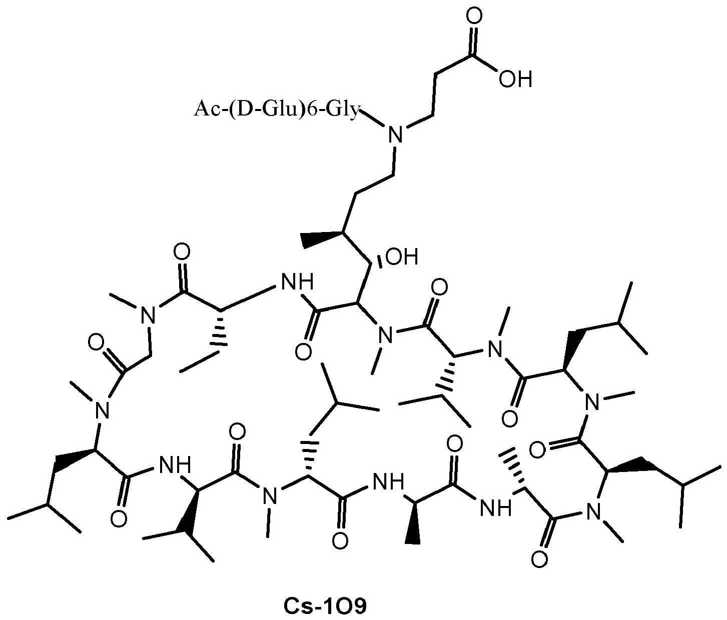

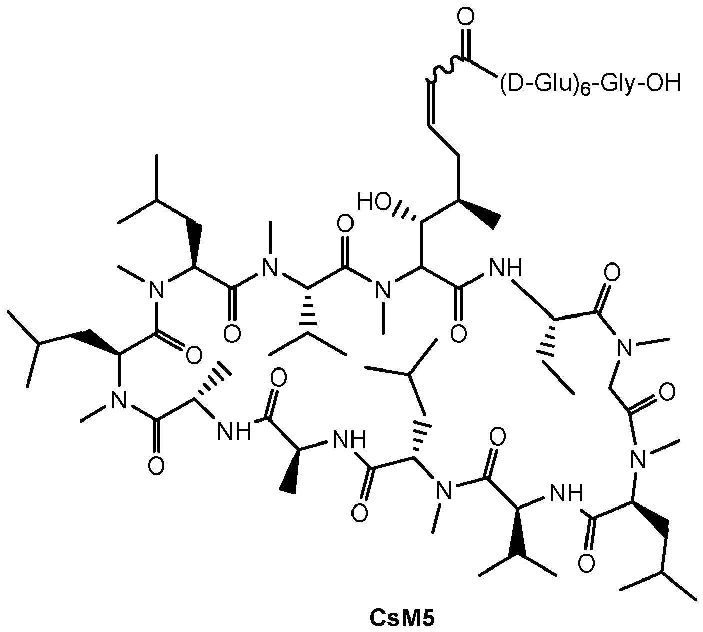

3. Cyclosporinderivat, ausgewählt aus den folgenden Verbindungen: 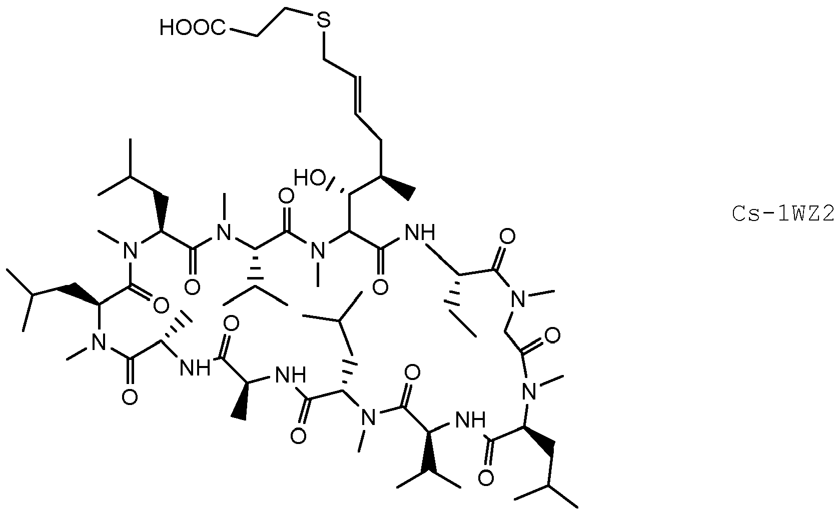 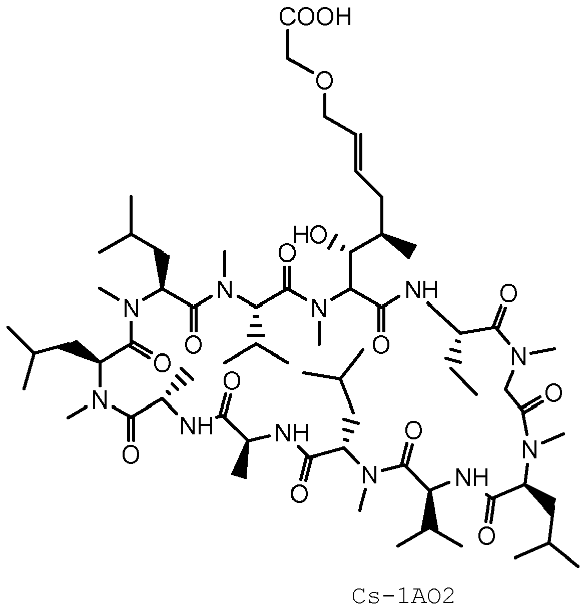 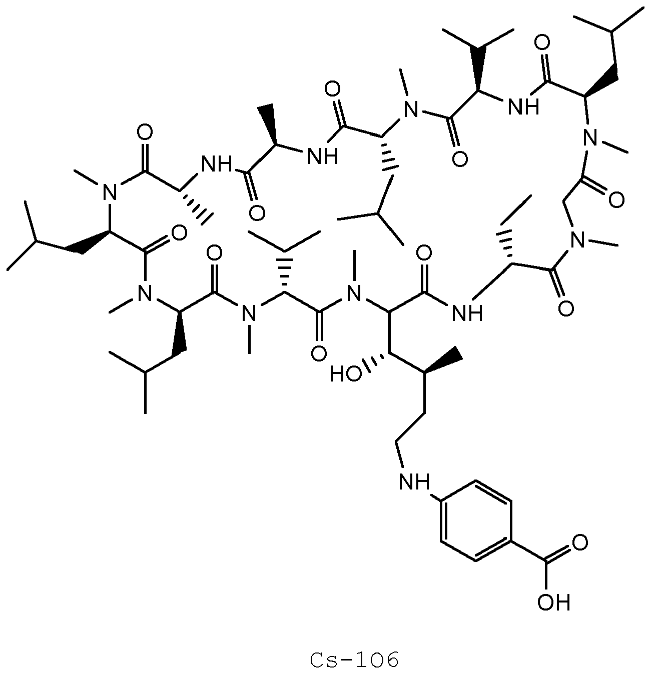 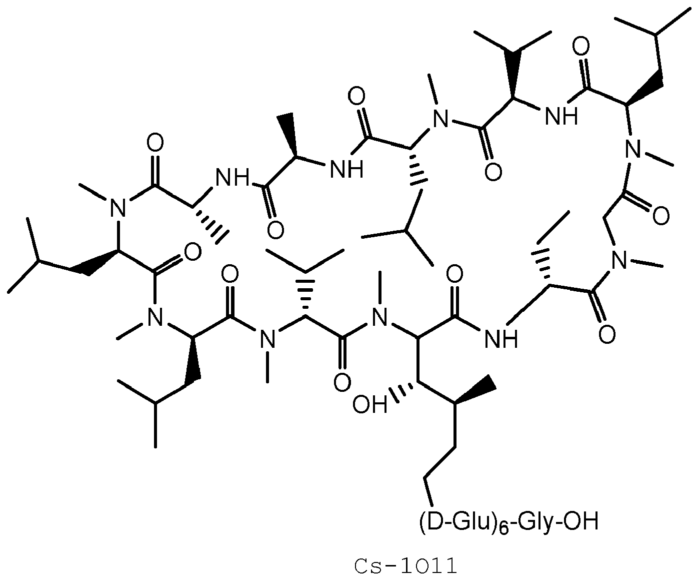 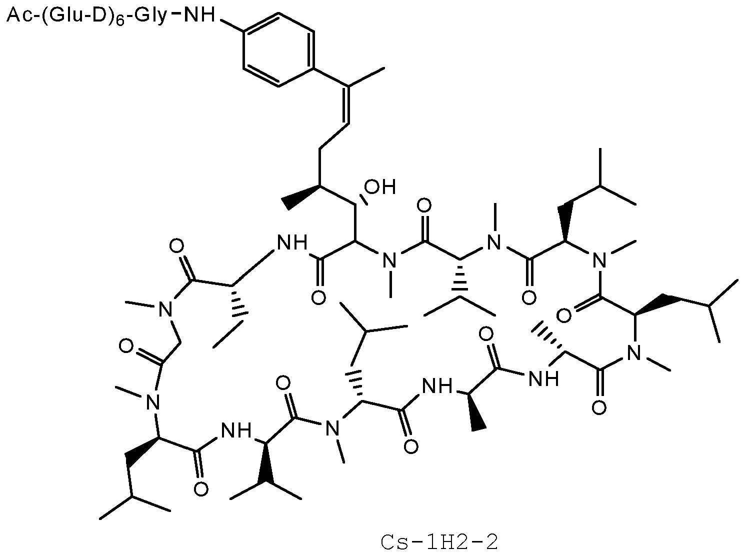 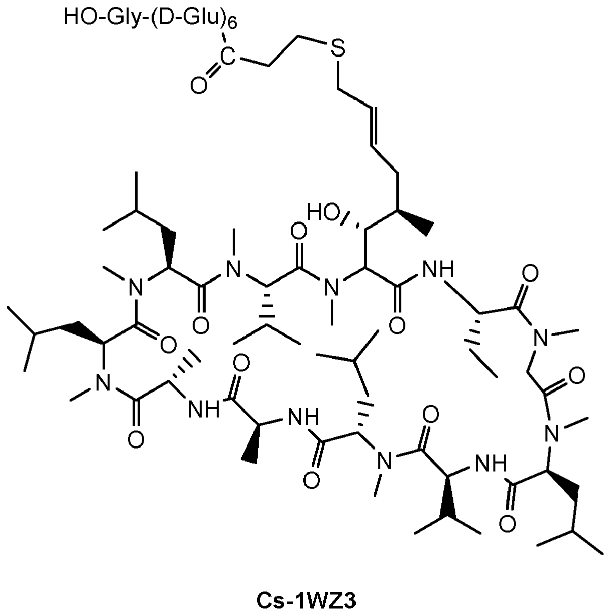 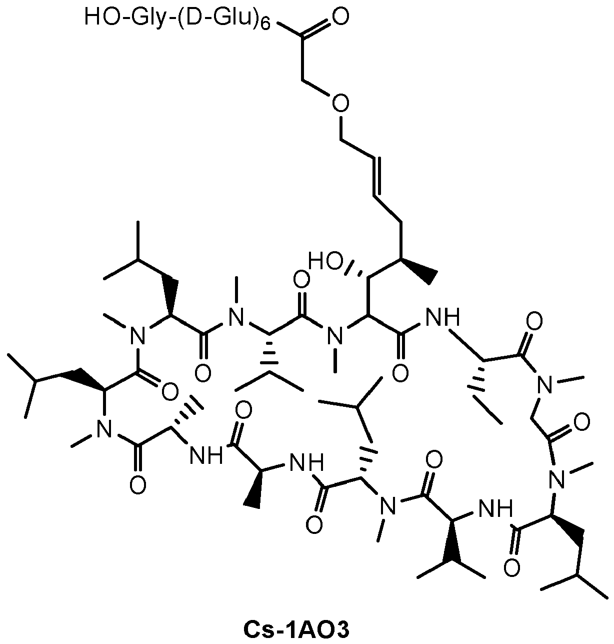 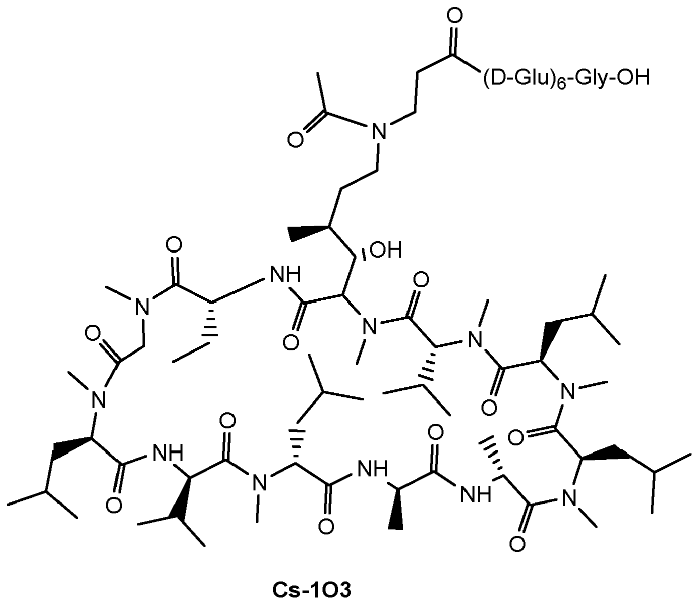 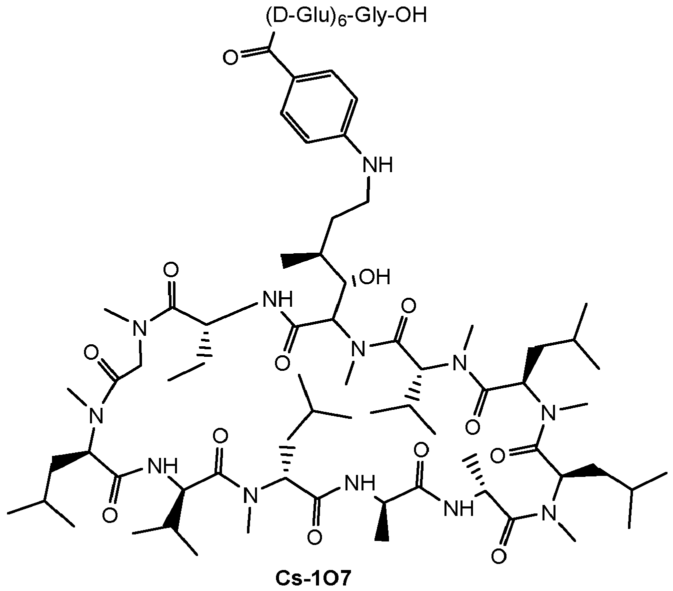 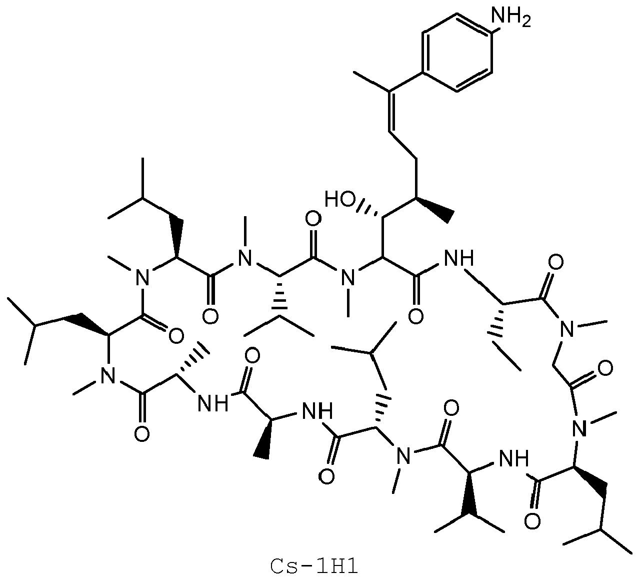 und

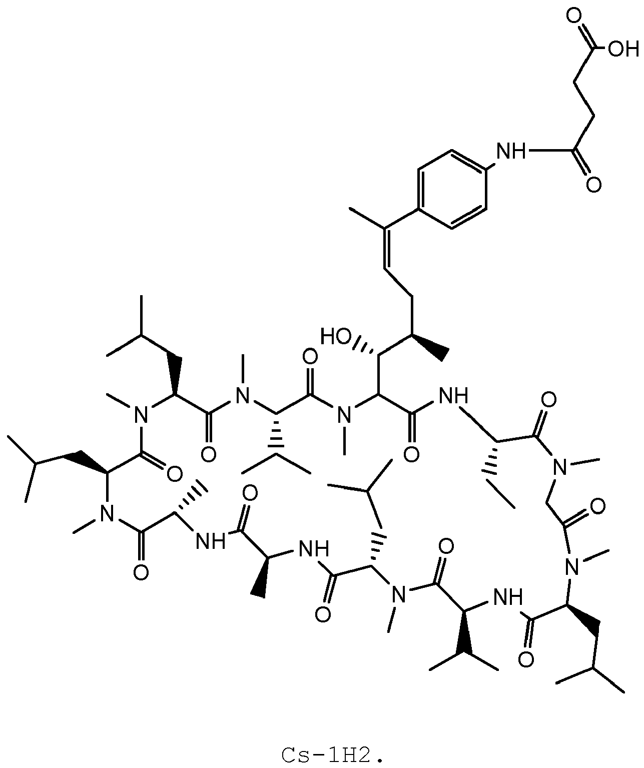

4. Verfahren zur Anreicherung eines Cyclosporinderivats in einem extrazellulären Raum eines multizellulären Objekts, umfassend die Schritte:
- Bereitstellen eines Cyclosporinderivats nach einem der Ansprüche 1 bis 3;
- Inkontaktbringen des Cyclosporinderivats mit einem multizellulären Objekt.

5. Verfahren nach Anspruch 4, **dadurch gekennzeichnet, dass** das multizelluläre Objekt ein separiertes Organ oder Körperteil, Blut oder eine Blutfraktion, eine Zellkultur oder eine Pflanze ist.

6. Verwendung eines Cyclosporinderivats nach einem der Ansprüche 1 bis 3 als Medikament.

7. Verwendung eines Cyclosporinderivats nach einem der Ansprüche 1 bis 3 zur Herstellung eines Medikaments zur Behandlung einer chronischen entzündlichen Erkrankung.

8. Verwendung nach Anspruch 7, wobei die chronische entzündliche Erkrankung Asthma, rheumatoide Arthritis, Multiple Sklerose, Psoriasis oder ulzerative Kolitis ist.

9. Pharmazeutische Zusammensetzung, umfassend ein Cyclosporinderivat nach einem der Ansprüche 1 bis 3.
